# EUROPÄISCHE PATENTSCHRIFT

(11) **EP 3 620 792 B1**
(45) Veröffentlichungstag und Bekanntmachung des Hinweises auf die Patenterteilung: **14.12.2022**
(21) Anmeldenummer: 19194596.3
(22) Anmeldetag: 30.08.2019
(51) Int. Cl.: G01N 33/50

(54) **VERFAHREN ZUR IN VITRO DIAGNOSE UND/ODER IN VITRO THERAPIEVERFOLGUNG EINER INFEKTION UND/ODER KRANKHEIT, INSBESONDERE INFEKTIONSKRANKHEIT, UND/ODER TUMORERKRANKUNG UND/ODER AUTOIMMUNERKRANKUNG UND/ODER ALLERGIE UND/ODER ZUR IMPFKONTROLLE UND/ODER ZUM NACHWEIS EINER KREUZPROTEKTIVITÄT SOWIE VERWENDUNG EINES KITS IN EINEM SOLCHEN VERFAHREN**
METHOD FOR IN VITRO DIAGNOSIS AND / OR IN VITRO THERAPY TRACKING OF INFECTION AND / OR DISEASE, IN PARTICULAR INFECTIOUS DISEASE, AND / OR TUMOUR DISEASE AND / OR AUTOIMMUNE DISEASE AND / OR ALLERGY AND / OR FOR VACCINATION CONTROL AND / OR FOR THE DETECTION OF CROSS-PROTECTION AND USE OF A KIT IN SUCH A METHOD
PROCÉDÉ DE DIAGNOSTIC IN VITRO ET / OU DE SUIVI DE THÉRAPIE IN VITRO D'UNE INFECTION ET / OU D'UNE MALADIE, EN PARTICULIER D'UNE MALADIE INFECTIEUSE, ET / OU D'UNE MALADIE TUMORALE ET / OU AUTO IMMUNE ET / OU D'UNE ALLERGIE ET / OU DE CONTRÔLE DE LA VACCINATION ET / OU DE VÉRIFICATION D'UNE PROTECTION CROISÉE AINSI QU'UTILISATION D'UN ENSEMBLE DANS UN TEL PROCÉDÉ

(30) Priorität: 31.08.2018 DE 102018214872
(43) Veröffentlichungstag der Anmeldung: 11.03.2020
(73) Patentinhaber: Genome Identification Diagnostics GmbH, 72479 Strassberg (DE)
(72) Erfinder: Preyer, Rosemarie, 72818 Trochtelfingen (DE)
(74) Vertreter: Patentanwälte Ruff, Wilhelm, Beier, Dauster & Partner mbB

(56) Entgegenhaltungen:
- US-A1- 2011 244 477
- ALEXANDROS HADJILAOU ET AL: "Single-Cell Analysis of B Cell/Antibody Cross-Reactivity Using a Novel Multicolor FluoroSpot Assay", THE JOURNAL OF IMMUNOLOGY, Bd. 195, Nr. 7, 28. August 2015 (2015-08-28), Seiten 3490-3496, XP055629965, ISSN: 0022-1767, DOI: 10.4049/jimmunol.1500918
- ADAM AWADALKAREEM ET AL: "Multiplexed FluoroSpot for the Analysis of Dengue Virus- and Zika Virus-Specific and Cross-Reactive Memory B Cells", JOURNAL OF IMMUNOLOGY, Bd. 201, Nr. 12, 9. November 2018 (2018-11-09), Seiten 3804-3814, XP009518112,

## Beschreibung

Die Erfindung betrifft ein Verfahren zur in vitro Diagnose und/oder in vitro Therapieverfolgung einer Infektion und/oder einer Krankheit, insbesondere Infektionskrankheit, und/oder einer Tumorerkrankung und/oder einer Autoimmunerkrankung und/oder einer Allergie und/oder zur Impfkontrolle und/oder zum Nachweis einer Kreuzprotektivität sowie die Verwendung eines Kits in einem solchen Verfahren.

Ein Verfahren zum Diagnostizieren einer Infektion, eines Tumors, einer Autoimmunerkrankung oder einer Allergie durch Detektieren von Antikörper sezernierenden Zellen ist aus der WO 2010/022980 A1 bekannt. Bei diesem Verfahren wird mittels einer Vielzahl unterschiedlicher Antigene, welche jeweils unterschiedlich markiert sind, überprüft, ob die Probe eines Subjekts Zellen enthält, welche Antikörper sezernieren. Dabei kann unter Umständen eine unspezifische Stimulation der Zellen vor dem Detektionsschritt erfolgen.

Ferner ist ein Immuno-Assay zur Diagnose einer Denguevirusinfektion sowie zur Überprüfung einer Serospezifität oder Kreuzprotektivität einer Antikörperreaktion auf eine Infektion mit dem Denguevirus bekannt, bei welchem festphasengebundene Denguevirus-spezifische Antikörper durch Zugabe von Dengueviren unterschiedlicher Serotypen sowie fluoreszenzmarkierten Detektionsantikörpern nachgewiesen werden (Alexandros Hadjilaou, J. Immunology, Bd. 195, Nr. 7, 28. August 2015, Seiten 3490-3496).

Bei Elispot-Verfahren, welche auf der Detektion von Antikörper sezernierenden Zellen beruhen, kann ein gewisses Risiko darin bestehen, dass aufgrund einer nur geringen Menge an sezernierten Antikörpern schlechte Signal-Hintergrund-Verhältnisse messbar sind, was das Risiko von falschnegativen und/oder falschpositiven Ergebnissen stark erhöht.

Eine unspezifische Stimulation hat insbesondere den Nachteil, dass manche Zellen spontan apoptotisch reagieren, d.h. einen programmierten Zelltod durchlaufen. Dies kann ebenfalls zu einer Verschlechterung von Signal-Rausch-Verhältnissen während eines Detektionsschrittes im Rahmen eines Elispot-Verfahrens führen. Ein weiterer Nachteil einer unspezifischen Stimulation besteht darin, dass generell Antikörper sezerniert werden, welche mit der Oberfläche eines Trägers eine Bindung eingehen und mit den spezifischen Antikörpern um freie Bindungsstellen konkurrieren, was ebenfalls zu Lasten der Sensitivität des Verfahrens geht.

### AUFGABE UND LÖSUNG

Die Erfindung stellt sich daher die Aufgabe, ein Verfahren bereitzustellen, welches Nachteile von konventionellen Verfahren, insbesondere konventionellen Elispot-Verfahren, welche auf einer Detektion von Antikörper sezernierenden Zellen und/oder von solchen Zellen sezernierten Antikörpern beruhen, umgeht.

Des Weiteren stellt sich die Erfindung die Aufgabe, die Verwendung eines Kits zur Durchführung eines solchen Verfahrens bereitzustellen.

Diese Aufgaben werden erfindungsgemäß gelöst durch ein Verfahren gemäß unabhängigem Anspruch 1 sowie durch ein Kit gemäß Anspruch 15. Bevorzugte Ausgestaltungen der Erfindung sind in den abhängigen Ansprüchen sowie in der Beschreibung definiert. Der Wortlaut sämtlicher Ansprüche wird hiermit durch Bezugnahme ausdrücklich zum Inhalt der Beschreibung gemacht.

Gemäß einem ersten Aspekt betrifft die Erfindung ein Verfahren zur in vitro Diagnose und/oder in vitro Therapieverfolgung einer Infektion und/oder einer Krankheit, insbesondere Infektionskrankheit, und/oder einer Tumorerkrankung und/oder einer Autoimmunerkrankung und/oder einer Allergie und/oder zur Impfkontrolle und/oder zum Nachweis einer Kreuzprotektivität.

Bei der Infektion und/oder Krankheit, insbesondere Infektionskrankheit, kann es sich insbesondere um eine Infektion und/oder Krankheit, insbesondere Infektionskrankheit, handeln, welche durch ein Pathogen verursacht wird, welches vorzugsweise ausgewählt ist aus der Gruppe bestehend aus Papillomaviridae, humane Papillomaviren, Herpesviridae, Herpes-simplex-Viren, Hepatitisviren, humane Immundefizienz-Viren, Influenzaviren, Parainfluenzaviren, Mumpsvirus, Masernvirus, Rötelnvirus, Parvovirus B19, Norovirus, Rotavirus, Polioviren, Coxsackieviren, Rhinoviren, Enteroviren, Adenoviren, Metapneumovirus, Mycobacterium-tuberculosis-Komplex, nichttuberkulöse Mykobakterien, Borrelien, Suptypen der genannten Pathogene und Kombinationen von wenigstens zwei der genannten Pathogene, insbesondere von wenigstens zwei Suptypen der genannten Pathogene.

Die humanen Papillomaviren können insbesondere ausgewählt sein aus der Gruppe bestehend aus HPV-6, HPV-11, HPV-16, HPV-18 und Kombinationen von wenigstens zwei der genannten humanen Papillomaviren.

Die Herpes-Simplex-Viren können insbesondere ausgewählt sein aus der Gruppe bestehend aus Herpes-simplex-Virus 1 (HHV-1), Herpes-simplex-Virus 2 (HHV-2), Varizella-Zoster-Virus (HHV-3), Epstein-Barr-Virus (HHV-4), humanes Cytomegalievirus (HHV-5), humanes Herpesvirus 6 (HHV-6), humanes Herpesvirus 7 (HHV-7), humanes Herpesvirus 8 (HHV-8) und Kombinationen von wenigstens zwei der genannten Herpes-Simplex-Viren.

Die Hepatitisviren können insbesondere ausgewählt sein aus der Gruppe bestehend aus Hepatitis-A-Virus, Hepatitis-B-Virus, Hepatitis-C-Virus, Hepatitis-D-Virus, Hepatitis-E-Virus und Kombinationen von wenigstens zwei der genannten Hepatitisviren.

Die humanen Immundefizienz-Viren können insbesondere ausgewählt sein aus der Gruppe bestehend aus HIV-1, HIV-2 und Kombinationen davon.

Die Influenzaviren können insbesondere ausgewählt sein aus der Gruppe bestehend aus Influenza-A-Viren, Influenza-B-Viren, Influenza-C-Viren und Kombinationen von wenigstens zwei der genannten Influenzaviren.

Die Parainfluenzaviren können insbesondere ausgewählt sein aus der Gruppe bestehend aus humanes Parainfluenzavirus Typ 1, humanes Parainfluenzavirus Typ 2, humanes Parainfluenzavirus Typ 3, humanes Parainfluenzavirus Typ 4 und Kombinationen von wenigstens zwei der genannten Parainfluenzaviren.

Die Polioviren können insbesondere ausgewählt sein aus der Gruppe bestehend aus Poliovirus Serotyp 1, Poliovirus Serotyp 2, Poliovirus Serotyp 3 und Kombinationen von wenigstens zwei der genannten Polioviren.

Die Coxsackieviren können insbesondere ausgewählt sein aus der Gruppe bestehend aus Coxsackievirus A, Coxsackievirus B und Kombinationen davon.

Die Rhinoviren können insbesondere ausgewählt sein aus der Gruppe bestehend aus Rhinovirus A, Rhinovirus B, Rhinovirus C und Kombinationen von wenigstens zwei der genannten Rhinoviren.

Die Enteroviren können insbesondere ausgewählt sein aus der Gruppe bestehend aus humanes Enterovirus 70, humanes Enterovirus 71 und Kombinationen davon.

Die Adenoviren können insbesondere ausgewählt sein aus der Gruppe bestehend aus humanes Adenovirus A (HAdV-A), humanes Adenovirus B (HAdV-B), humanes Adenovirus C (HAdV-C), humanes Adenovirus D (HAdV-D), humanes Adenovirus E (HAdV-E), humanes Adenovirus F (HAdV-F) und Kombinationen von wenigstens zwei der genannten Adenoviren.

Der Mycobacterium-tuberculosis-Komplex kann insbesondere ausgewählt sein aus der Gruppe bestehend aus Mycobacterium tuberculosis, Mycobacterium bovis, Mycobacterium africanum, Mycobacterium microti und Kombinationen von wenigstens zwei der genannten Mykobakterien.

Die nichttuberkulösen Mykobakterien können insbesondere ausgewählt sein aus der Gruppe bestehend aus Mycobacterium kansasii, Mycobacterium marinum, Mycobacterium scrofulaceum, Mycobacterium malmoense, Mycobacterium avium, Mycobacterium intracellulare, Mycobacterium chelonae, Mycobacterium fortuitum, Mycobacterium xenopi, Mycobacterium ulcerans, Mycobacterium abscessus und Kombinationen von wenigstens zwei der genannten nichttuberkulösen Mykobakterien.

Die Borrelien können insbesondere ausgewählt sein aus der Gruppe bestehend aus Borrelia burgdorferi, Borrelia afzelii, Borrelia garinii, Borrelia spielmanii, Borrelia miyamotoi und Kombinationen von wenigstens zwei der genannten Borrelien.

Die Tumorerkrankung, bei welcher es sich insbesondere um eine Karzinomerkrankung handeln kann, kann ausgewählt sein aus der Gruppe bestehend aus Gebärmutterhalstumor, Kaposi-Sarkom, Melanom, Lymphom, Leukämie, Prostatakarzinom, Lungenkarzinom, Brustkrebs, Darmkrebs und Hals-Rachenkarzinom.

Die Autoimmunerkrankung kann ausgewählt sein aus der Gruppe bestehend aus Erkrankungen des entzündlich-rheumatischen Formenkreises, Multiple Sklerose, Colitis Ulcerosa und Hashimoto-Thyreoiditis.

Beispielsweise kann die Krankheit ausgewählt sein aus der Gruppe bestehend aus Gebärmutterhalskrebs, Kopf-Hals-Karzinomen, Lippenherpes, Genitalherpes, Mundfäule, Herpes-simplex-Enzephalitis, Windpocken, Gürtelrose, Pfeiffersches Drüsenfieber, Zytomegalie, Drei-Tage-Fieber, Kaposi-Sarkom, Hepatits A, Hepatits B, Hepatitis C, Hepatitis D, Hepatitis E, AIDS, Influenza, Parainfluenza, Mumps, Masern, Röteln, Ringelröteln, Poliomyelitis, Tuberkulose, Borreliose, virale Meningitis, virale Myokarditis, Schnupfen, Erkältungen und Durchfall.

Die Allergie kann ausgewählt sein aus der Gruppe bestehend aus Lebensmittelallergie, Hausstauballergie, Insektengiftallergie, Gräserallergie, Baumpollenallergie, Arzneimittelallergie und Latexallergie.

Das erfindungsgemäße Verfahren weist die folgenden Schritte auf:
(a) Bereitstellen
   - einer Probe, welche Antikörper sezernierende Zellen enthält,
   - eines Trägers mit einer Oberfläche, an welcher Anti-Ig-Antikörper immobilisiert sind, und
   - einer Vielzahl unterschiedlicher Antigene aus verschiedenen oder unterschiedlichen infektiösen Pathogenen, insbesondere aus verschiedenen oder unterschiedlichen Subtypen von infektiösen Pathogenen, und/oder aus verschiedenen oder unterschiedlichen Tumorantigenen und/oder aus verschiedenen oder unterschiedlichen Autoimmun-Antigenen und/oder aus verschiedenen oder unterschiedlichen Allergenen, wobei die Antigene jeweils unterschiedlich markiert sind,
(b) Inkontaktbringen der Trägeroberfläche, d.h. der Oberfläche des Trägers, mit der Vielzahl unterschiedlicher Antigene, d.h. mit der gemäß Schritt (a) bereitgestellten Vielzahl unterschiedlicher Antigene, sowie der Probe unter Bedingungen, welche dazu geeignet sind, dass die Antikörper sezernierenden Zellen durch die Antigene stimuliert werden und Antikörper, welche durch die stimulierten Antikörper sezernierenden Zellen produziert werden, an die immobilisierten Anti-Ig-Antikörper der Trägeroberfläche binden und die Antigene an Antikörper binden, welche für die Antigene spezifisch sind, und
(c) Detektieren von markierten Antigenen, welche an der Trägeroberfläche abgefangen wurden, wodurch die An- oder Abwesenheit von in der Probe enthaltenen Antikörper sezernierenden Zellen, welche für die markierten Antigene spezifisch sind, detektiert wird.

Insbesondere können/kann der Schritt (a) zeitlich vor dem Schritt (b) und/oder der Schritt (b) zeitlich vor dem Schritt (c) durchgeführt werden. Insbesondere können die Schritte (a) bis (c) zeitlich aufeinanderfolgend, d.h. zeitlich hintereinander oder zeitlich nacheinander, durchgeführt werden. Insbesondere kann der Schritt (a) zeitlich vor dem Schritt (b) und der Schritt (b) zeitlich vor dem Schritt (c) durchgeführt werden. Ferner können die Schritte (a) bis (c) insbesondere unmittelbar aufeinanderfolgend, d.h. unmittelbar hintereinander oder unmittelbar nacheinander, durchgeführt werden. Mit anderen Worten kann der Schritt (b) zeitlich unmittelbar nach dem Schritt (a) und der Schritt (c) zeitlich unmittelbar nach dem Schritt (b) durchgeführt werden.

Durch die Anwesenheit, insbesondere die Anzahl, von Antikörper sezernierenden Zellen in der Probe und/oder die Abwesenheit von Antikörper sezernierenden Zellen in der Probe kann eine Infektion und/oder Krankheit, insbesondere Infektionskrankheit, und/oder Tumorerkrankung und/oder Autoimmunerkrankung und/oder Allergie diagnostiziert und/oder eine Therapie oder ein Therapieverlauf einer Infektion und/oder Krankheit, insbesondere Infektionskrankheit, und/oder Tumorerkrankung und/oder Autoimmunerkrankung und/oder Allergie verfolgt werden.

Weiterhin kann durch die Anwesenheit, insbesondere die Anzahl, von Antikörper sezernierenden Zellen in der Probe und/oder die Abwesenheit von Antikörper sezernierenden Zellen in der Probe der Erfolg einer Impfung nachgewiesen, d.h. eine Impfung kontrolliert, werden.

Weiterhin kann durch die Anwesenheit, insbesondere die Anzahl, von Antikörper sezernierenden Zellen in der Probe und/oder die Abwesenheit von Antikörper sezernierenden Zellen in der Probe, insbesondere bei Verwendung unterschiedlich markierter Antigene aus verschiedenen Subtypen eines Pathogens, mit besonderem Vorteil nachgewiesen oder überprüft werden, ob ein Patient eine Kreuzprotektivität aufweist.

Das erfindungsgemäße Verfahren stellt ein Elispot-Verfahren zur in vitro Diagnose und/oder in vitro Therapieverfolgung einer Infektion und/oder einer Krankheit, insbesondere Infektionskrankheit, und/oder einer Tumorerkrankung und/oder einer Autoimmunerkrankung und/oder einer Allergie und/oder zur Impfkontrolle und/oder zum Nachweis einer Kreuzprotektivität dar. Das Verfahren beruht auf der Detektion bzw. dem Nachweis von Antikörper sezernierenden Zellen und/oder von ihnen sezernierten Antikörpern, wobei die Antikörper sezernierenden Zellen mit einer Vielzahl unterschiedlicher Antigene, welche jeweils unterschiedlich markiert sind, inkubiert bzw. stimuliert (spezifische Stimulation) und anschließend die Antikörper sezernierenden Zellen und/oder sezernierte Antikörper davon mit Hilfe der Antigene, detektiert werden. Mit anderen Worten wird somit bei dem erfindungsgemäßen Verfahren - im Gegensatz zum dem in der WO 2010/022980 A1 beschriebenen Verfahren - die Vielzahl der unterschiedlichen Antigene sowohl zur spezifischen Stimulation von Antikörper sezernierenden Zellen als auch zu deren Detektion (Nachweis) und/oder zur Detektion (Nachweis) von Antikörpern, welche von den Antikörper sezernierenden Zellen sezerniert werden, verwendet.

Durch die spezifische Stimulation erfolgt im Ergebnis hauptsächlich eine Sezernierung und anschließende Immobilisierung von antigenspezifischen Antikörpern. Eine Immobilisierung unspezifischer Antikörper an die Oberfläche des Trägers wird mithin vermieden, jedenfalls überwiegend. Dies führt zu deutlich besseren Detektionsergebnissen aufgrund ausreichend freier Bindungsstellen, die nicht von Antikörper unspezifisch induzierter Stimulation blockiert werden. Das verbessert signifikant das Signal-Hintergrund-Verhältnis. Dadurch kann das Risiko von falschnegativen und/oder falschpositiven Ergebnissen beträchtlich gesenkt werden.

Gegenüber dem in der WO 2010/022980 A1 beschriebenen Verfahren besteht ein weiterer Vorteil darin, dass im Zusammenhang einer unspezifischen Stimulation mit nachfolgendem Wasch- und Inkubationsschritt anfallende Verfahrensschritte entfallen oder entbehrlich sind. Dies spiegelt mit besonderem Vorteil die in einem menschlichen Organismus oder nichtmenschlichen Säugetierorganismus ablaufenden Vorgänge besser wider, was ebenfalls zu einer erhöhten Spezifität des erfindungsgemäßen Verfahrens beiträgt.

Dadurch, dass weiterhin eine Vielzahl unterschiedlich markierter Antigene zur Inkubation bzw. Stimulation der Antikörper sezernierenden Zellen sowie gleichzeitig zur Detektion der Antikörper sezernierenden Zellen und/oder sezernierten Antikörpern davon verwendet wird, können in einem Reaktionsansatz, beispielsweise in einer Vertiefung (sogenanntes Well) einer Mikrotiterplatte, eine Vielzahl unterschiedlicher Infektionen und/oder unterschiedlicher Krankheiten, insbesondere Infektionskrankheiten, und/oder unterschiedlicher Tumorerkrankungen und/oder unterschiedlicher Autoimmunerkrankungen und/oder unterschiedlicher Allergien diagnostiziert und/oder die Therapie oder der Therapieverlauf einer Vielzahl unterschiedlicher Infektionen und/oder unterschiedlicher Krankheiten, insbesondere Infektionskrankheiten, und/oder unterschiedlicher Tumorerkrankungen und/oder unterschiedlicher Autoimmunerkrankungen und/oder unterschiedlicher Allergien verfolgt werden.

Unter dem Ausdruck "Infektion" soll im Sinne der vorliegenden Erfindung das aktive oder passive Eindringen, Verbleiben und anschließende Vermehren von Pathogenen in einem Organismus, insbesondere in einem menschlichen Organismus oder in einem nicht menschlichen Säugetierorganismus, verstanden werden.

Unter dem Ausdruck "Infektionskrankheit" soll im Sinne der vorliegenden Erfindung eine durch Pathogene hervorgerufene Erkrankung, insbesondere in einem menschlichen Organismus oder in einem nicht menschlichen Säugetierorganismus, verstanden werden.

Unter dem Ausdruck "Pathogene" sollen im Sinne der vorliegenden Erfindung vorzugsweise Krankheitserreger, insbesondere Bakterien und/oder Viren und/oder Pilze und/oder Parasiten, verstanden werden.

Unter dem im Zusammenhang eines Pathogens verwendeten Ausdruck "Subtypen" soll im Sinne der vorliegenden Erfindung verschiedene Unterarten und/oder Nebenarten und/oder Ausprägungen und/oder Gruppen, wie beispielsweise "low Risk"-Gruppen und/oder "high Risk"-Gruppen, eines Pathogens und/oder Autoimmun-Antigens und/oder Tumorantigens und/oder tuberkulöse Mykobakterien und/oder nicht-tuberkulöse Mykobakterien verstanden werden.

Unter dem Ausdruck "Kreuzprotektivität" soll im Sinne der vorliegenden Erfindung eine schützende Immunreaktion auf nicht in einem verwendeten Impfstoff enthaltene Subtypen durch identische oder ähnliche Oberflächenstrukturen verstanden werden.

Als Probe kann grundsätzlich eine Probe menschlichen Ursprungs oder nichtmenschlichen Ursprungs, insbesondere equinen, bovinen, porcinen, leporiden oder murinen Ursprungs, verwendet werden. Bevorzugt handelt es sich bei der Probe um eine menschliche Probe.

Als Träger wird bevorzugt eine Mikrotiterplatte, beispielsweise eine Mikrotiterplatte mit 96 oder 384 Vertiefungen (sogenannte Wells), und/oder eine oder mehrere Vertiefungen einer Mikrotiterplatte, beispielsweise einer Mikrotiterplatte mit 96 oder 384 Vertiefungen, verwendet.

Als Anti-Ig-Antikörper wird vorzugsweise ein Anti-lgG-Antikörper, ein Anti-IgM-Antikörper oder ein Anti-lgA-Antikörper verwendet. Besonders bevorzugt wird ein Anti-lgG-Antikörper verwendet. Bevorzugt handelt es sich bei dem Anti-Ig-Antikörper um einen Antihuman-Ig-Antikörper, insbesondere um einen Antihuman-IgG-Antikörper, Antihuman-IgM-Antikörper oder einen Antihuman-IgA-Antikörper. Insbesondere bevorzugt ist ein Antihuman-IgG-Antikörper.

In Ausgestaltung der Erfindung wird beim Durchführen von Schritt (b) zunächst die Trägeroberfläche mit der Vielzahl unterschiedlicher Antigene in Kontakt gebracht und anschließend die Probe zu der Trägeroberfläche und der Vielzahl unterschiedlicher Antigene hinzugegeben. Dadurch können mit besonderem Vorteil unerwünschte Zelladhärierungen an einer Wandung des Trägers vermieden und eine homogene Verteilung der Zellen erzielt werden.

Grundsätzlich kann beim Durchführen von Schritt (b) jedoch auch zunächst die Trägeroberfläche mit der Probe in Kontakt gebracht und anschließend die Vielzahl unterschiedlicher Antigene zu der Trägeroberfläche und der Probe hinzugegeben werden.

In weiterer Ausgestaltung der Erfindung wird als Probe eine Probe verwendet, welche mononukleäre Zellen, insbesondere B-Zellen, einer Körperflüssigkeit, insbesondere des Blutes, des peripheren Blutes, des Liquors oder der Synovialflüssigkeit, und/oder eines Körpergewebes, insbesondere eines gesunden Gewebes, wie beispielsweise eines Lymphgewebes, oder eines krankhaft veränderten Gewebes, wie beispielsweise eines Tumorgewebes, enthält.

Bevorzugt wird als Probe eine Probe verwendet, welche mononukleäre Zellen, insbesondere B-Zellen, des peripheren Bluts enthält.

In weiterer Ausgestaltung der Erfindung werden die mononukleären Zellen vor Durchführen von Schritt (b) isoliert. Die Isolierung der mononukleären Zellen erfolgt vorzugsweise mittels Dichtegradientenzentrifugation oder mittels magnetischer Zellseparation (Magnetic Activated Cell Sorting, MACS). Besonders bevorzugt werden vor dem Durchführen von Schritt (b) mononukleäre Zellen, insbesondere B-Zellen, aus Blut/peripherem Blut mittels Dichtegradientenzentrifugation oder mittels magnetischer Zellseparation isoliert. Durch die in diesem Absatz beschriebenen Verfahrensmaßnahmen können mit besonderem Vorteil Probenbestandteile, insbesondere Blutbestandteile, abgetrennt werden, welche den Detektionsschritt (c) erschweren oder gar unmöglich machen würden.

In weiterer Ausgestaltung der Erfindung werden zellgebundene und/oder membranständige, d. h. membrangebundene, Antikörper, insbesondere B-Zell-Rezeptoren, der mononukleären Zellen, insbesondere B-Zellen, vor Durchführen von Schritt (b) entfernt. Dadurch ist mit besonderem Vorteil eine zusätzliche Verbesserung des Signal-Hintergrund-Verhältnisses und mithin eine Verbesserung des Detektionsschritts (c) erzielbar, wodurch das Risiko von falschnegativen und/oder falschpositiven Ergebnissen zusätzlich minimiert werden kann.

In weiterer Ausgestaltung der Erfindung werden zum Entfernen der zellmembrangebundenen Antikörper, insbesondere B-Zell-Rezeptoren, folgende Schritte durchgeführt:
- Inkubieren der mononukleären Zellen, insbesondere der B-Zellen, in einem Zellkulturmedium, bevorzugt über einen Zeitraum von 10 min bis 60 min, insbesondere 20 min bis 40 min, bevorzugt 30 min, und/oder bei einer Temperatur von 20 °C bis 40 °C, insbesondere 30 °C bis 40 °C, bevorzugt 37 °C,
- Zentrifugieren der inkubierten mononukleären Zellen, insbesondere B-Zellen, und des Zellkulturmediums, bevorzugt mit einer Rotationsgeschwindigkeit von 400 × g bis 1000 × g, insbesondere 700 × g, und/oder über einen Zeitraum von 5 min bis 15 min, insbesondere 10 min, und/oder bei einer Temperatur von 18 °C bis 25 °C, insbesondere 20 °C,
- Abtrennen des Zellkulturmediums nach dem Zentrifugieren unter Erhalt eines Zellpellets,
- Resuspendieren des Zellpellets und Inkubieren der Zellen in einem weiteren, vorzugsweise gleichen Zellkulturmedium, bevorzugt über einen Zeitraum von 5 min bis 15 min, insbesondere 10 min, und/oder bei einer Temperatur von 30 °C bis 40 °C, insbesondere 37 °C,
- Zentrifugieren der inkubierten Zellen und des weiteren, vorzugsweise gleichen, Zellkulturmediums, bevorzugt mit einer Rotationsgeschwindigkeit von 400 × g bis 1000 × g, insbesondere 700 × g, und/oder über einen Zeitraum von 5 min bis 15 min, insbesondere 10 min, und/oder bei einer Temperatur von 18 °C bis 25 °C, insbesondere 20 °C, und
- Abtrennen des weiteren, vorzugsweise gleichen, Zellkulturmediums unter Erhalt eines weiteren Zellpellets, wobei Zellen des weiteren Zellpellets eine reduzierte Anzahl an membranständigen Antikörpern, insbesondere B-Zell-Rezeptoren, aufweisen.

Durch die im vorherigen Absatz beschriebene Ausgestaltung der Erfindung ist eine zusätzliche Optimierung des Signal-Hintergrund-Verhältnisses und mithin des Detektionsschrittes (c) erzielbar, wodurch im Ergebnis das Risiko von falschnegativen und/oder falschpositiven Ergebnissen zusätzlich reduziert werden kann.

Als Zellkulturmedium kann beispielsweise ein Zellkulturmedium verwendet werden, welches ausgewählt ist aus der Gruppe bestehend aus RPMI-1640, AIM-V, CTL-Test, CTS OpTmizer T Cell Expansion SFM und X-VIVO. Die vorgenannten Zellkulturmedien sind besonders für die Kultivierung mononukleärer Zellen geeignet.

In weiterer Ausgestaltung der Erfindung wird als Vielzahl unterschiedlicher Antigene eine Vielzahl unterschiedlicher Antigene verwendet, wobei die Antigene jeweils mit unterschiedlichen Farbstoffen, insbesondere unterschiedlichen Fluoreszenzfarbstoffen, vorzugsweise ausgewählt aus der Gruppe bestehend aus Cyanine, Fluorescein, Rhodamine, Curamine und Xanthene, markiert sind.

In weiterer Ausgestaltung der Erfindung wird der Schritt (b) über einen Zeitraum von 30 Minuten bis 5 Tagen, insbesondere 30 Minuten bis 48 Stunden, bevorzugt 30 Minuten bis 24 Stunden, durchgeführt. Wird der Schritt (b) über einen Zeitraum von 30 Minuten bis 48 Stunden, insbesondere 30 Minuten bis 24 Stunden, durchgeführt, können mit besonderem Vorteil ausschließlich Plasmazellen detektiert bzw. nachgewiesen werden, da Gedächtnis-B-Zellen während dieser Zeiträume, insbesondere während eines Zeitraums von 30 Minuten bis 24 Stunden, nicht aktiviert werden können. Sofern zusätzlich die Detektion bzw. der Nachweis von Gedächtnis-B-Zellen erwünscht ist, kann der Schritt (b) über einen längeren Zeitraum, beispielsweise über einen Zeitraum länger als 48 Stunden, insbesondere über einen Zeitraum von 3 Tagen bis 5 Tagen, durchgeführt werden. Sofern lediglich die Detektion bzw. der Nachweis von Gedächtnis-B-Zellen gewünscht ist, kann dies durch Messung über einen Zeitraum von 30 Minuten bis 48 Stunden, insbesondere 30 Minuten bis 24 Stunden, und durch eine weitere Messung über einen Zeitraum länger als 48 Stunden, insbesondere über einen Zeitraum von 3 Tagen bis 5 Tagen, sowie durch anschließende Differenzbildung der erhaltenen Messergebnisse erfolgen.

In weiterer Ausgestaltung der Erfindung wird der Schritt (b) bei einer Temperatur von 20 °C bis 40 °C, insbesondere 30 °C bis 40 °C, bevorzugt 35 °C bis 40 °C, besonders bevorzugt bei ca. 37 °C, durchgeführt.

In weiterer Ausgestaltung der Erfindung werden die Antigene beim Durchführen von Schritt (c) durch das Vorliegen eines Spots (Fleck), insbesondere eines farbigen oder fluoreszierenden Spots, oder einer Vielzahl von Spots (Flecken), insbesondere einer Vielzahl von farbigen oder fluoreszierenden Spots, auf der Trägeroberfläche detektiert, wodurch die An- oder Abwesenheit von in der Probe enthaltenen Antikörper sezernierenden Zellen, welche für die Antigene spezifisch sind, und/oder von Antikörpern, welche von den Antikörper sezernierenden Zellen sezerniert werden, detektiert wird. Zur Detektion, insbesondere Sichtbarmachung, des bzw. der Spots können grundsätzlich mit alkalischer Phosphatase oder Meerrettichperoxidase konjugierte Antikörper verwendet werden. Alternativ können als Detektionsantikörper mit Biotin konjugierte Antikörper in Kombination mit Streptavidin, welches mit alkalischer Phosphatase konjugiert ist, oder mit Biotin konjugierte Antikörper in Kombination mit Streptavidin, welches mit Meerrettichperoxidase konjugiert ist, verwendet werden. Die Detektion beruht in all diesen Fällen auf einer durch das Enzym (alkalische Phosphatase oder Meerrettichperoxidase) katalysierten Umsetzung eines farberzeugenden Substrats, wie beispielsweise 3-Amino-9-ethylcarbazol (AEC), 4-Chlor-1-naphthol (CN), 3,3'-Diaminobenzidin (DAB), Tetramethylbenzidin (TMB), 5-Brom-4-chlor-3-indoxylphosphat (BCIP) oder Nitroblautetrazoliumchlorid (NBT).

In weiterer Ausgestaltung der Erfindung werden als Vielzahl von Pathogenen unterschiedliche Subtypen desselben Pathogens verwendet.

In weiterer Ausgestaltung der Erfindung werden als Vielzahl unterschiedlicher Antigene Varianten desselben Antigens verwendet.

In weiterer Ausgestaltung der Erfindung wird die Vielzahl unterschiedlicher Antigene ausgewählt aus verschiedenen oder unterschiedlichen infektiösen Pathogenen, welche ausgewählt sind aus der Gruppe bestehend aus Papillomaviridae, humane Papillomaviren, Herpesviridae, Herpes-simplex-Viren, Hepatitisviren, humane Immundefizienz-Viren, Influenzaviren, Parainfluenzaviren, Mumpsvirus, Masernvirus, Rötelnvirus, Parvovirus B19, Norovirus, Rotavirus, Polioviren, Coxsackieviren, Rhinoviren, Enteroviren, Adenoviren, Metapneumovirus, Mycobacterium-tuberculosis-Komplex, nichttuberkulöse Mykobakterien, Borrelien, Suptypen der genannten Pathogene und Kombinationen von wenigstens zwei der genannten Pathogene, insbesondere von wenigstens zwei Suptypen der genannten Pathogene.

Die humanen Papillomaviren können insbesondere ausgewählt sein aus der Gruppe bestehend aus HPV-6, HPV-11, HPV-16, HPV-18 und Kombinationen von wenigstens zwei der genannten humanen Papillomaviren.

Die Herpes-Simplex-Viren können insbesondere ausgewählt sein aus der Gruppe bestehend aus Herpes-simplex-Virus 1 (HHV-1), Herpes-simplex-Virus 2 (HHV-2), Varizella-Zoster-Virus (HHV-3), Epstein-Barr-Virus (HHV-4), humanes Cytomegalievirus (HHV-5), humanes Herpesvirus 6 (HHV-6), humanes Herpesvirus 7 (HHV-7), humanes Herpesvirus 8 (HHV-8) und Kombinationen von wenigstens zwei der genannten Herpes-Simplex-Viren.

Die Hepatitisviren können insbesondere ausgewählt sein aus der Gruppe bestehend aus Hepatitis-A-Virus, Hepatitis-B-Virus, Hepatitis-C-Virus, Hepatitis-D-Virus, Hepatitis-E-Virus und Kombinationen von wenigstens zwei der genannten Hepatitisviren.

Die humanen Immundefizienz-Viren können insbesondere ausgewählt sein aus der Gruppe bestehend aus HIV-1, HIV-2 und Kombinationen davon.

Die Influenzaviren können insbesondere ausgewählt sein aus der Gruppe bestehend aus Influenza-A-Viren, Influenza-B-Viren, Influenza-C-Viren und Kombinationen von wenigstens zwei der genannten Influenzaviren.

Die Parainfluenzaviren können insbesondere ausgewählt sein aus der Gruppe bestehend aus humanes Parainfluenzavirus Typ 1, humanes Parainfluenzavirus Typ 2, humanes Parainfluenzavirus Typ 3, humanes Parainfluenzavirus Typ 4 und Kombinationen von wenigstens zwei der genannten Parainfluenzaviren.

Die Polioviren können insbesondere ausgewählt sein aus der Gruppe bestehend aus Poliovirus Serotyp 1, Poliovirus Serotyp 2, Poliovirus Serotyp 3 und Kombinationen von wenigstens zwei der genannten Polioviren.

Die Coxsackieviren können insbesondere ausgewählt sein aus der Gruppe bestehend aus Coxsackievirus A, Coxsackievirus B und Kombinationen davon.

Die Rhinoviren können insbesondere ausgewählt sein aus der Gruppe bestehend aus Rhinovirus A, Rhinovirus B, Rhinovirus C und Kombinationen von wenigstens zwei der genannten Rhinoviren.

Die Enteroviren können insbesondere ausgewählt sein aus der Gruppe bestehend aus humanes Enterovirus 70, humanes Enterovirus 71 und Kombinationen davon.

Die Adenoviren können insbesondere ausgewählt sein aus der Gruppe bestehend aus humanes Adenovirus A (HAdV-A), humanes Adenovirus B (HAdV-B), humanes Adenovirus C (HAdV-C), humanes Adenovirus D (HAdV-D), humanes Adenovirus E (HAdV-E), humanes Adenovirus F (HAdV-F) und Kombinationen von wenigstens zwei der genannten Adenoviren.

Der Mycobacterium-tuberculosis-Komplex kann insbesondere ausgewählt sein aus der Gruppe bestehend aus Mycobacterium tuberculosis, Mycobacterium bovis, Mycobacterium africanum, Mycobacterium microti und Kombinationen von wenigstens zwei der genannten Mykobakterien.

Die nichttuberkulösen Mykobakterien können insbesondere ausgewählt sein aus der Gruppe bestehend aus Mycobacterium kansasii, Mycobacterium marinum, Mycobacterium scrofulaceum, Mycobacterium malmoense, Mycobacterium avium, Mycobacterium intracellulare, Mycobacterium chelonae, Mycobacterium fortuitum, Mycobacterium xenopi, Mycobacterium ulcerans, Mycobacterium abscessus und Kombinationen von wenigstens zwei der genannten nichttuberkulösen Mykobakterien.

Die Borrelien können insbesondere ausgewählt sein aus der Gruppe bestehend aus Borrelia burgdorferi, Borrelia afzelii, Borrelia garinii, Borrelia spielmanii, Borrelia miyamotoi und Kombinationen von wenigstens zwei der genannten Borrelien.

Insbesondere kann die Vielzahl unterschiedlicher Antigene aus unterschiedlichen oder verschiedenen Subtypen humaner Papillomaviren (HPV-Subtypen) ausgewählt werden, insbesondere aus der Gruppe bestehend aus HPV 6, HPV 11, HPV 16, HPV 18, HPV 31, HPV 33, HPV 35, HPV 39, HPV 45, HPV 51, HPV 52, HPV 56, HPV 58, HPV 68, HPV 73 und HPV 82.

Weiterhin kann die Vielzahl unterschiedlicher Antigene aus unterschiedlichen oder verschiedenen humanen "low-risk" Papillomaviren-Subtypen ("low-risk"-HPV-Subtypen) ausgewählt werden, insbesondere aus der Gruppe bestehend aus HPV 6, HPV 11, HPV 40, HPV 42, HPV 43, HPV 44, HPV 53, HPV 54, HPV 61, HPV 72, HPV 73 und HPV 81.

Weiterhin kann die Vielzahl unterschiedlicher Antigene aus unterschiedlichen oder verschiedenen humanen "high-risk" Papillomaviren-Subtypen ("high-risk"-HPV-Subtypen) ausgewählt werden, insbesondere aus der Gruppe bestehend aus HPV 16, HPV 18, HPV 31, HPV 33, HPV 35, HPV 39, HPV 45, HPV 51, HPV 52, HPV 56, HPV 58, HPV 59, HPV 68, HPV 73 und HPV 82, bevorzugt aus der Gruppe bestehend aus HPV 16, HPV 18, HPV 31, HPV 33, HPV 45, HPV 52 und HPV 58.

Die verschiedenen oder unterschiedlichen Tumorantigene können ausgewählt werden aus der Gruppe bestehend aus MART-1, Melan-A, p97, beta-HCG, GalNAc, MAGE-1, MAGE-2, MAGE-4, MAGE-12, MUC1, MUC2, MUC3, MUC4, MUC18, CEA, DDC, P1A, EpCam, Melanomantigen gp75 Hker 8, hochmolekulares Molanomantigen, K19, Tyr1, Tyr2, Mitglieder der pMel 17 Genfamilie, cMet, PSA (Prostataantigen), PSM (prostate mucin antigen), PSMA (prostate specific membrane antigen), Prostata sekretorisches Protein, alpha-1-Fetoprotein, CA125, CA19.9, TAG-72, BRCA-1 und BRCA-2.

Die verschiedenen oder unterschiedlichen Autoimmun-Antigene können ausgewählt werden aus der Gruppe bestehend aus Insulin, Glutamatdecarboxylase 65 (GAD 65), Hitzeschockprotein 60 (HSP 60), basisches Myelinprotein (MBP), Myelin-Oligodendrozyten-Protein (MOG), Proteolipid-Protein (PLP), und Collagentyp II.

Die verschiedenen oder unterschiedlichen Allergene können ausgewählt werden aus der Gruppe bestehend aus Insektengiften, Gräserpollen, Baumpollen und Hausstaubmilben.

In weiterer Ausgestaltung der Erfindung werden die Antigene, insbesondere der Spot bzw. die Vielzahl von Spots, beim Durchführen von Schritt (c) mittels Lichtmikroskopie, Fluoreszenzmikroskopie oder mittels eines Fluoreszenz-Messgeräts, wie beispielsweise eines Fluorospot Readers, detektiert. Ein geeignetes Fluoreszenz-Messgerät wird von der Anmelderin beispielsweise unter der Bezeichnung AID iSpot (ELR08IFL), AID iSpot Spectrum (ELR088IFL), AID iSpot Robot (ELROB07IFL), AID vSpot (VSR07), AID vSpot Spectrum (VSR078IFL) sowie AID multiSpot (MSR08) kommerziell vertrieben.

In weiterer Ausgestaltung der Erfindung wird ferner als Positivkontrolle die Gesamtzahl der in der Probe enthaltenen Antikörper sezernierenden Zellen gemessen. Dadurch lässt sich mit besonderem Vorteil feststellen, ob die Probe überhaupt vitale, d.h. insbesondere zur Sezernierung von Antikörpern befähigte, Zellen aufweist und das Verfahren unter Verwendung der Probe überhaupt zu auswertbaren Ergebnissen führt. Der Erhalt von falschnegativen Ergebnissen kann dadurch verhindert werden. Weiterhin hat die Durchführung einer Positivkontrolle den Vorteil, dass patientenspezifische Umstände, wie beispielsweise eine bestimmte Medikation oder immunologische Erkrankungen, erfasst werden können.

Die Positivkontrolle wird vorzugsweise mittels eines gegen den Isotyp der sezernierten Antikörper gerichteten Detektionsantikörpers und/oder mittels eines Antigens, vorzugsweise mittels Mitogene, wie beispielsweise Pokeweed Mitogen, gemessen. Als Detektionsantikörper zur Sichtbarmachung von Spots kann grundsätzlich ein mit alkalischer Phosphatase oder Meerrettichperoxidase konjugierter Antikörper und/oder ein fluoreszenzmarkierter Antikörper verwendet werden. Alternativ kann als Detektionsantikörper ein mit Biotin konjugierter Antikörper in Kombination mit alkalischer Phosphatase, welche mit Streptavidin konjugiert ist, oder ein mit Biotin konjugierter Antikörper in Kombination mit Meerrettichperoxidase, welche mit Streptavidin konjugiert ist, verwendet werden. Die Detektion beruht in all diesen Fällen auf einer durch das Enzym (alkalische Phosphatase oder Meerrettichperoxidase) katalysierten Umsetzung eines farberzeugenden Substrats, wie beispielsweise 2,2'-Azino-di-(3-ethylbenzthiazolin-6-sulfonsäure) (ABTS), 3-Amino-9-ethylcarbazol (AEC), 4-Chlor-1-naphthol (CN), 3,3'-Diaminobenzidin (DAB), Tetramethylbenzidin (TMB), 5-Brom-4-chlor-3-indoxylphosphat (BCIP) oder Nitroblautetrazoliumchlorid (NBT).

In weiterer Ausgestaltung der Erfindung wird als Negativkontrolle ferner in Abwesenheit der Antigene eine Produktion (sogenannte spontane Produktion) von Antikörpern durch die Antikörper sezernierenden Zellen in einem Zellkulturmedium gemessen. Dadurch kann mit besonderem Vorteil eine unspezifische Stimulation der Zellen, beispielsweise infolge eines Aufreinigungsprozesses oder anderer äußerer Einflüsse, erkannt und somit falschpositive Ergebnisse vermieden werden. Bezüglich geeigneter Zellkulturmedien wird vollständig auf die in der bisherigen Beschreibung offenbarten Zellkulturmedien Bezug genommen.

In weiterer Ausgestaltung der Erfindung werden ferner als Negativkontrolle markierte Antigene in Abwesenheit von Antikörper sezernierenden Zellen in einem Zellkulturmedium gemessen. Dadurch kann mit besonderem Vorteil überprüft werden, ob und gegebenenfalls in welchem Umfang in Abwesenheit von Antikörper sezernierenden Zellen unspezifische Bindungen der markierten Antigene an der Oberfläche des Trägers auftreten. Durch die Messung einer Negativkontrolle kann mit besonderem Vorteil der Erhalt von falschpositiven Ergebnissen verhindert werden. Bezüglich geeigneter Zellkulturmedien wird vollständig auf die in der bisherigen Beschreibung offenbarten Zellkulturmedien Bezug genommen.

Gemäß einem zweiten Aspekt betrifft die Erfindung die Verwendung eines Kits zur Durchführung eines Verfahrens gemäß erstem Erfindungsaspekt.

Das Kit weist räumlich voneinander getrennt Folgendes auf:
- einen Träger mit einer Oberfläche, an welcher Anti-Ig-Antikörper immobilisiert sind, und
- eine Vielzahl unterschiedlicher Antigene aus verschiedenen oder unterschiedlichen infektiösen Pathogenen und/oder verschiedenen oder unterschiedlichen Tumorantigenen und/oder verschiedenen oder unterschiedlichen Autoimmun-Antigenen und/oder verschiedenen oder unterschiedlichen Allergenen, wobei die Antigene jeweils unterschiedlich markiert sind.

Alternativ weist das Kit räumlich voneinander getrennt Folgendes auf:
- einen Träger mit einer Oberfläche,
- Anti-Ig-Antikörper zur Immobilisierung an einer Oberfläche des Trägers und
- eine Vielzahl unterschiedlicher Antigene aus verschiedenen oder unterschiedlichen infektiösen Pathogenen und/oder aus verschiedenen oder unterschiedlichen Tumorantigenen und/oder aus verschiedenen oder unterschiedlichen Autoimmun-Antigenen und/oder aus verschiedenen oder unterschiedlichen Allergenen, wobei die Antigene jeweils unterschiedlich markiert sind.

Alternativ weist das Kit räumlich voneinander getrennt Folgendes auf:
- einen Träger mit einer Oberfläche und
- Anti-Ig-Antikörper zur Immobilisierung an einer Oberfläche des Trägers.

Alternativ weist das Kit räumlich voneinander getrennt Folgendes auf:
- einen Träger mit einer Oberfläche und
- eine Vielzahl unterschiedlicher Antigene aus verschiedenen oder unterschiedlichen infektiösen Pathogenen und/oder aus verschiedenen oder unterschiedlichen Tumorantigenen und/oder aus verschiedenen oder unterschiedlichen Autoimmun-Antigenen und/oder aus verschiedenen oder unterschiedlichen Allergenen, wobei die Antigene jeweils unterschiedlich markiert sind.

Alternativ weist das Kit räumlich voneinander getrennt Folgendes auf:
- Anti-Ig-Antikörper zur Immobilisierung an einer Oberfläche eines Trägers und
- eine Vielzahl unterschiedlicher Antigene aus verschiedenen oder unterschiedlichen infektiösen Pathogenen und/oder aus verschiedenen oder unterschiedlichen Tumorantigenen und/oder aus verschiedenen oder unterschiedlichen Autoimmun-Antigenen und/oder aus verschiedenen oder unterschiedlichen Allergenen, wobei die Antigene jeweils unterschiedlich markiert sind.

Des Weiteren kann der Kit wenigstens eine Komponente aufweisen, welche ausgewählt ist aus der Gruppe bestehend aus Fluoreszenzmessgerät wie Elispot/Fluorospot Reader System, Mikrotiterplatten, Fluoreszenzverstärker, Mitogene, Detektionsantikörper, farbstofferzeugende Enzymsubstrate, Pufferlösungen, Waschlösungen und Kombinationen von wenigstens zwei der genannten Komponenten.

Bezüglich weiterer Merkmale und Vorteile des Kits wird zur Vermeidung von Wiederholungen vollständig auf die im Rahmen des ersten Erfindungsaspekts gemachten Ausführungen Bezug genommen. Die dort insbesondere in Bezug auf das Verfahren beschriebenen Merkmale und Vorteile gelten sinngemäß auch für das Kit gemäß zweitem Erfindungsaspekt.

Weitere Merkmale und Vorteile der Erfindung ergeben sich aus der nachfolgenden Beschreibung von bevorzugten Ausführungsformen in Form von Beispielen. Dabei können einzelne Merkmale jeweils für sich alleine oder in Kombination miteinander verwirklicht sein. Die nachfolgend beschriebenen Beispiele dienen lediglich der weiteren Erläuterung der Erfindung, ohne die Erfindung auf den Offenbarungsgehalt der Beispiele zu beschränken.

### FIGURENKURZBESCHREIBUNGEN

In den Figuren ist Folgendes schematisch gezeigt:
- Fig. 1a - e:: eine Ausführungsform eines erfindungsgemäßen Verfahrens,
- Fig. 2:: ein Pipettierschema eines Ansatzes eines erfindungsgemäßen HPV-B-Zell-Verfahrens,
- Fig. 3a-b:: Beispiele zweier Vorversuche zur Findung einer geeigneten Antigen- und Zellkonzentration eines geimpften und eines nicht geimpften Spenders,
- Fig. 4a-f:: Beispielergebnisse dreier geimpfter und dreier nicht geimpfter Spender im HPV-B-Zell-Elispot,
- Fig. 5a-d:: Zusammenfassung der Ergebnisse von 6 geimpften und 15 nicht geimpften Spendern vergleichend aufgetragen in einem Box-Whisker-Plot, HPV Typen einzeln aufgetragen und
- Fig 6a-b:: Zusammenfassung der Ergebnisse von 6 geimpften und 15 nicht geimpften Spendern vergleichend aufgetragen in einem Box-Whiskeor-Plot, HPV VLP Mix.

### AUSFÜHRLICHE FIGURENBESCHREIBUNG

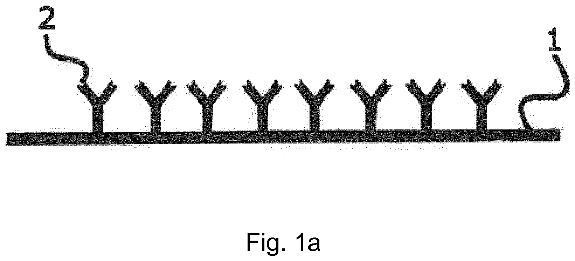

In den Figuren 1a-e ist schematisch der Ablauf einer Ausführungsform eines erfindungsgemäßen Verfahrens wiedergegeben.

Gemäß Figur 1a wird unter anderem ein Träger 1 mit einer Oberfläche bereitgestellt, an welcher Anti-Ig-Antikörper 2 immobilisiert sind. Bei den Anti-Ig-Antikörpern handelt es sich vorzugsweise um Antihuman-IgG-Antikörper. Als Träger 1 kann beispielsweise die Vertiefung (Well) einer Mikrotiterplatte verwendet werden.

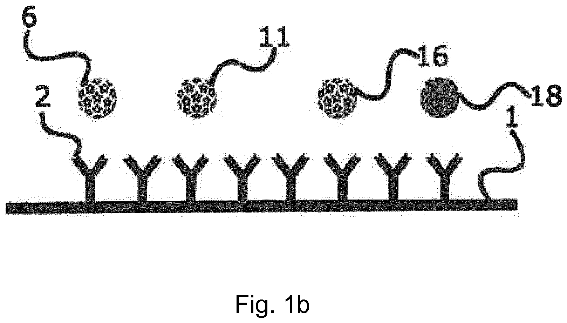

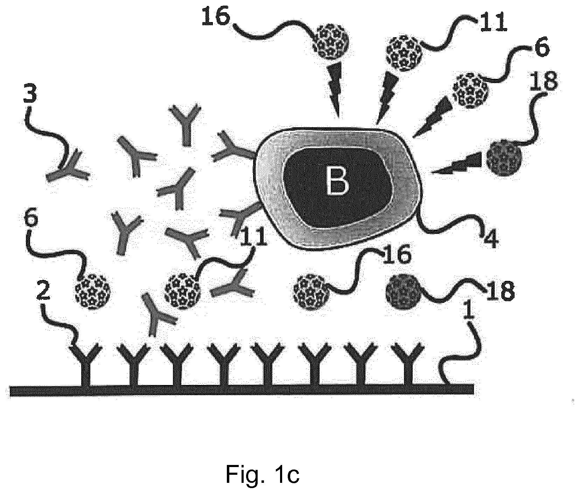

In einem nächsten Schritt wird die Oberfläche des Trägers 1 mit einer Vielzahl unterschiedlicher Antigene 6, 11, 16 und 18 sowie mit einer Probe, welche Antikörper sezernierende Zellen enthält, in Kontakt gebracht (siehe Fig. 1b und Fig. 1c). Die Vielzahl unterschiedlicher Antigene kann dabei aus verschiedenen oder unterschiedlichen infektiösen Pathogenen, insbesondere aus verschiedenen oder unterschiedlichen Subtypen davon, stammen. Beispielsweise kann es sich bei dem Antigen 6 um HPV 6 (humanes Papillomavirus-Subtyp 6), bei dem Antigen 11 um HPV 11 (humanes Papillomavirus-Subtyp 11), bei dem Antigen 16 um HPV 16 (humanes Papillomavirus-Subtyp 16) und bei dem Antigen 18 um HPV 18 (humanes Papillomavirus-Subtyp 18) handeln. Alternativ oder in Kombination kann es sich bei den unterschiedlichen Antigenen um unterschiedliche Tumorantigene und/oder unterschiedliche Autoimmun-Antigene und/oder unterschiedliche Allergene handeln.

Die unterschiedlichen Antigene 6, 11, 16 und 18 sind jeweils unterschiedlich markiert, beispielsweise mit unterschiedlichen Fluoreszenzfarbstoffen.

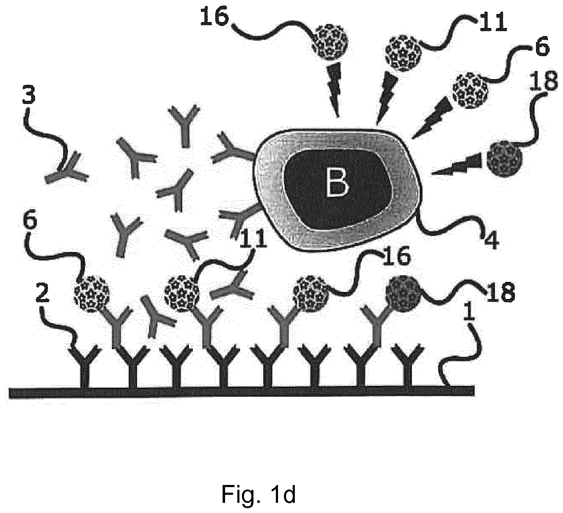

Das Inkontaktbringen der Oberfläche des Trägers 1 mit der Vielzahl unterschiedlicher Antigene 6, 11, 16 und 18 erfolgt unter Bedingungen, welche dazu geeignet sind, dass Antikörper 3, welche durch die Antikörper sezernierenden Zellen 4 gegen die Antigene 6, 11, 16 und 18 produziert werden, an die immobilisierten Anti-Ig-Antikörper 2 binden (siehe Figuren 1c und 1d).

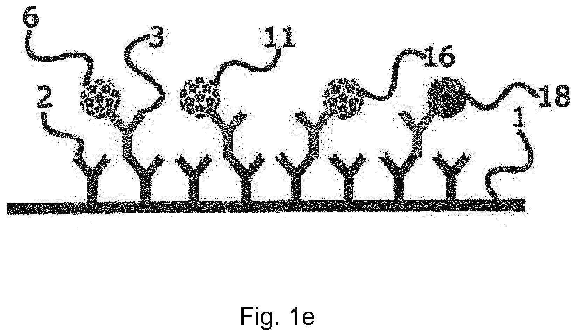

Nach Entfernung der Antikörper sezernierenden Zellen 4 sowie der nichtgebundenen markierten Antigene und nicht gebundenen Antikörper, insbesondere mittels Waschschritten, kann eine Detektion der markierten und auf der Oberfläche des Trägers 1 (über die immobilisierten Anti-Ig-Antikörper 2) abgefangenen Antigene 6, 11, 16 und 18 vorgenommen und mithin die An- oder Abwesenheit von antigenspezifischen Antikörper sezernierenden Zellen 4 in der Probe, vorzugsweise durch Fluoreszenzmessungen, detektiert werden (siehe Fig. 1e).

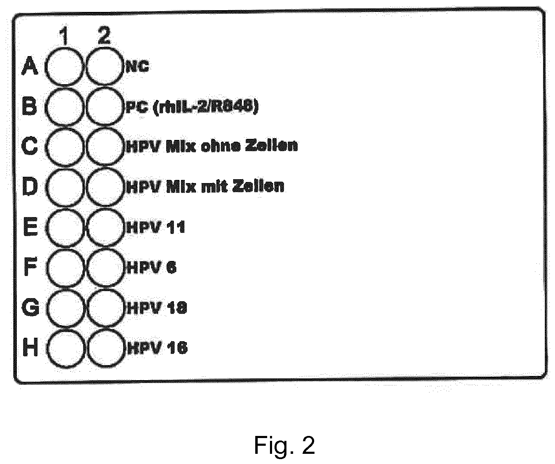

Figur 2 zeigt schematisch ein Pipettierschema eines Ansatzes eines erfindungsgemäßen HPV-B-Zell-Elispots zum Nachweis antigenspezifischer B-Lymphozyten mittels der Sekretion von IgG.

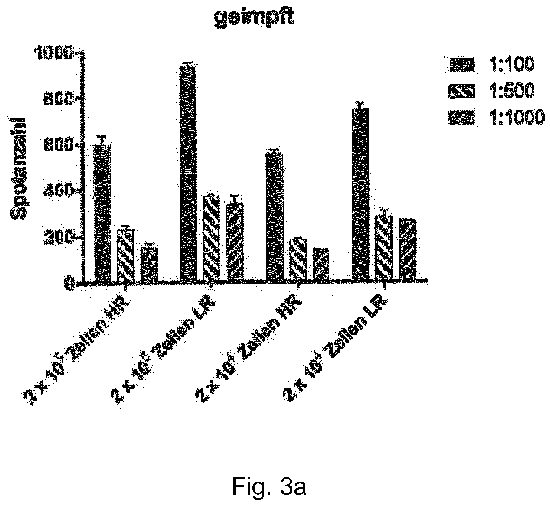

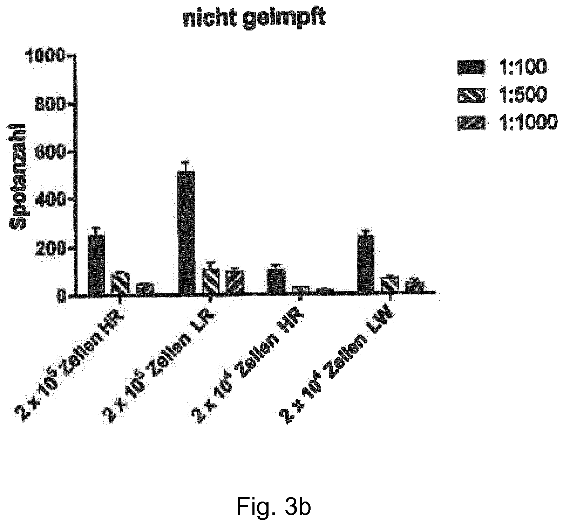

Die Figuren 3a und b zeigen grafisch die Ergebnisse eines Vorversuchs zur Bestimmung einer geeigneten Zellkonzentration (2×10⁵ Zellen / Well und 2×10⁴ Zellen / Well) und einer optimalen Verdünnungsstufe (1:100, 1:500 und 1:1000) der VLPs. Exemplarisch sind die Ergebnisse des VLP Mix eines geimpften (Fig. 3a) und eines nicht geimpften Spenders (Fig. 3b) dargestellt. Es wurde für jeden Spender ein Doppelansatz je Zellzahl und Verdünnungsstufe angesetzt. Aufgetragen sind jeweils der arithmetische Mittelwert des Doppelansatzes und die dazugehörige Standardabweichung. Die high-Risk HPV-Typen 16 und 18 wurden mit einem grünen Farbstoff (Alexa Fluor 488) markiert, während die low-Risk Typen 11 und 6 mit einem roten Farbstoff (Alexa Fluor 555) markiert wurden.

Bei einer Verdünnung der VLPs im Verhältnis 1:100 konnten deutlich erhöhte Signale beobachtet werden. Dies zeigte sich sowohl bei geimpften und nicht geimpften Spendern, was für eine unspezifische Reaktion spricht. Allerdings konnten nur sehr geringe Unterschiede in der Spotanzahl zwischen den Verdünnungsstufen 1:500 und 1:1000 beobachtet werden. Bei der Betrachtung der zugehörigen Bilder fällt jedoch auf, dass bei einer Verdünnung von 1:500 das Fluoreszenzsignal deutlich stärker ist, sowie die Form und Größe der Spots besser den optimalen Charakteristika eines Spots entsprechen.

Generell können nur sehr geringe Unterschiede zwischen 2×10⁵ Zellen / Well und 2×10⁴ Zellen / Well beobachtet werden. Bei der Betrachtung der zugehörigen Bilder fällt jedoch auf, dass eine homogenere Verteilung der Zellen bei 2×10⁵ Zellen / Well zu beobachten ist.

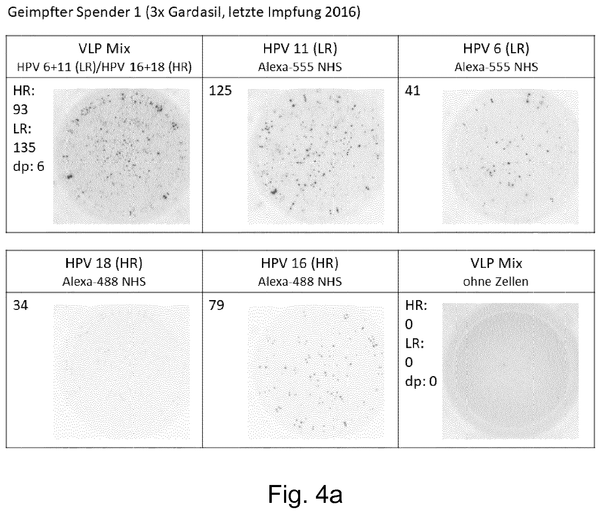

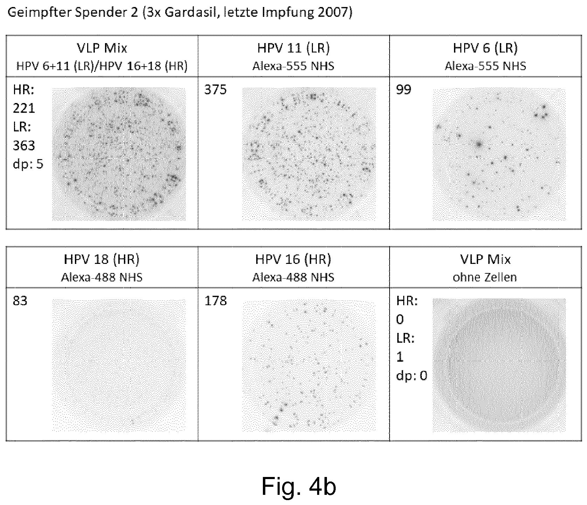

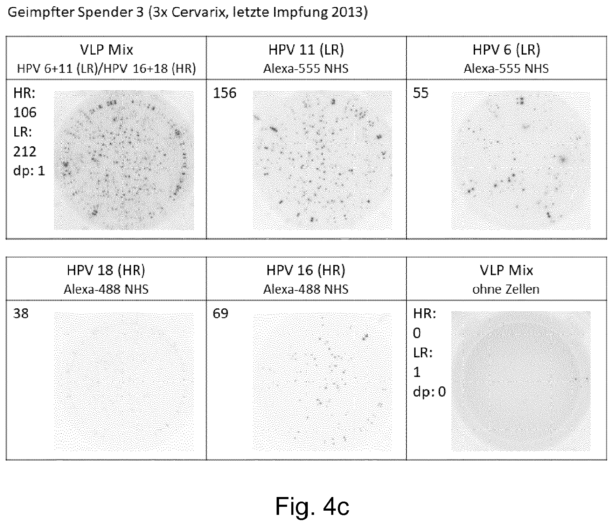

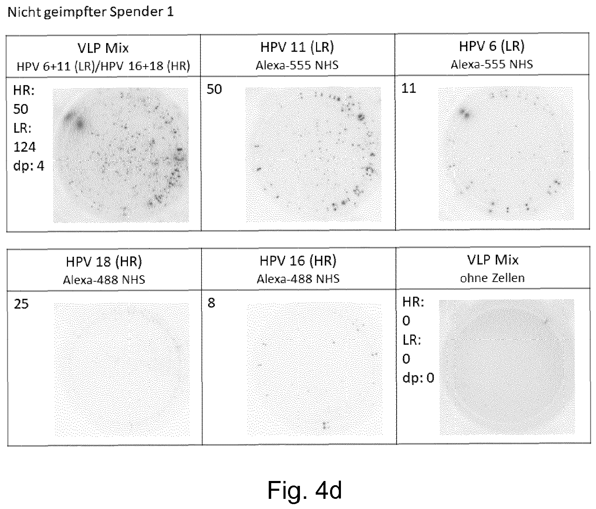

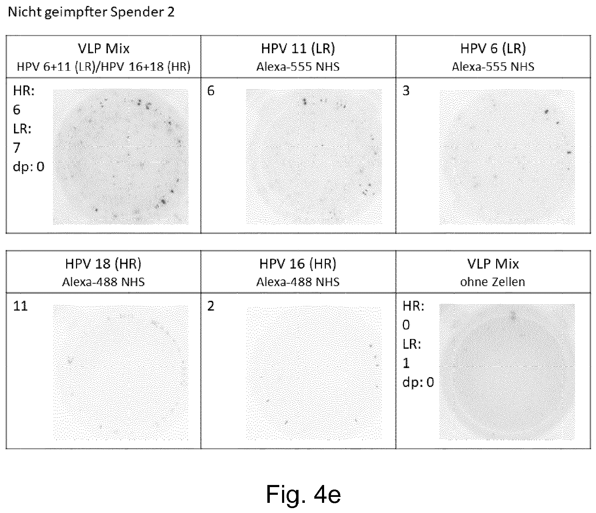

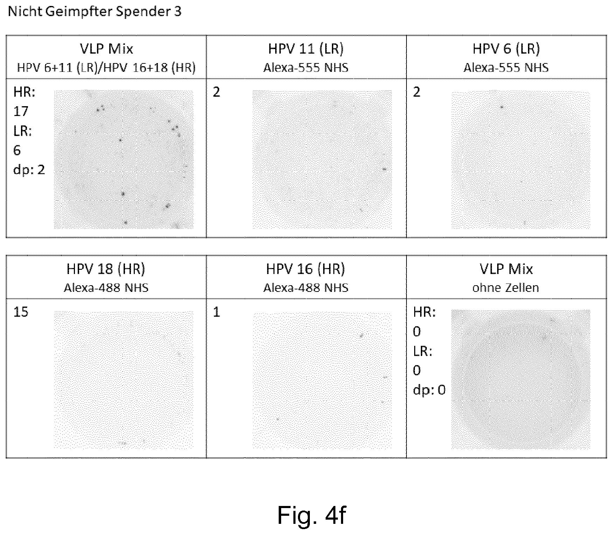

Die Figuren 4a-f zeigen repräsentative Beispiele des HPV-B-Zell-Elispots dreier geimpfter (Fig. 4a-c) und dreier nicht geimpfter (Fig. 4d-f) Spender. Dargestellt sind Assays, welche mit einer Zellkonzentration von 2×10⁵ Zellen / Well und der VLP Verdünnungsstufe 1:500 angesetzt wurden. Das Pipettierschema für den B-Zell Elispot entspricht dem in Fig. 2 dargestellten Schema.

Das Bild oben links zeigt jeweils ein überlagertes Bild des HPV VLP Mix (FITC-Kanal + Cy3-Kanal) mit den zugehörigen Zählwerten der low-Risk (LR) und high-Risk (HR) Typen. Darüber hinaus ist auch die Anzahl doppelt positiver (dp) Spots angegeben und somit der Anteil kreuzprotektiver B-Zellen, welche sowohl low-Risk als auch high-Risk HPV Typen erkennen können. Das Bild oben in der Mitte zeigt jeweils die Ergebnisse der HPV 11 (LR) VLPs im Cy3-Kanal. Das Bild oben rechts zeigt jeweils die Ergebnisse der HPV 6 (LR) VLPs ebenfalls im Cy3-Kanal. Das Bild unten links zeigt jeweils die Ergebnisse der HPV 18 (HR) VLPs im FITC-Kanal. Das Bild unten in der Mitte zeigt jeweils die Ergebnisse der HPV 16 (HR) VLPs ebenfalls im FITC-Kanal. Und letztlich das Bild unten rechts, welches jeweils ein überlagertes Bild des HPV VLP Mix (FITC-Kanal + Cy3-Kanal), welches ohne Zellen inkubiert wurde, zeigt. Diese Kontrolle dokumentiert, dass es zu keiner unspezifischen Bindung der markierten VLPs kam.

Links einer jeden Abbildung ist die von einem Elispot Reader ermittelte Spotanzahl angegeben. Zur besseren Visualisierung wurden sämtliche Fluoreszenzbilder in Schwarz-Weiß-Bilder umgewandelt und davon jeweils das Negativ erstellt.

Aus den Figuren 4a-f geht ein eindeutiger Unterschied sowohl im Erscheinungsbild als auch in der Anzahl gezählter Spots zwischen den einzelnen Spendern und Spendergruppen hervor. Die Ergebnisse der antigenstimulierten Wells und die zugehörigen Kontrollen zeigen deutlich, dass es sich bei dem gewählten Versuchsansatz um ein valides Verfahren handelt, welches eine antigenspezifische Untersuchung von B-Zellen auf Einzelzellebene ermöglicht.

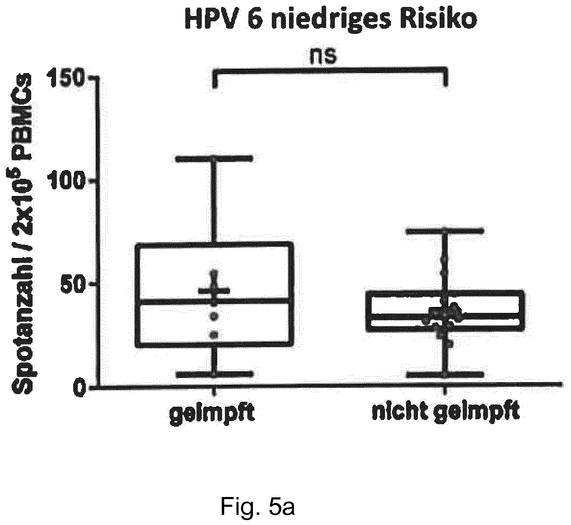

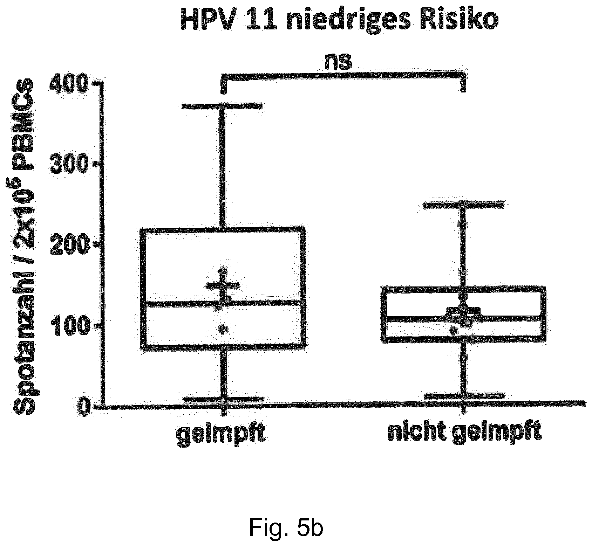

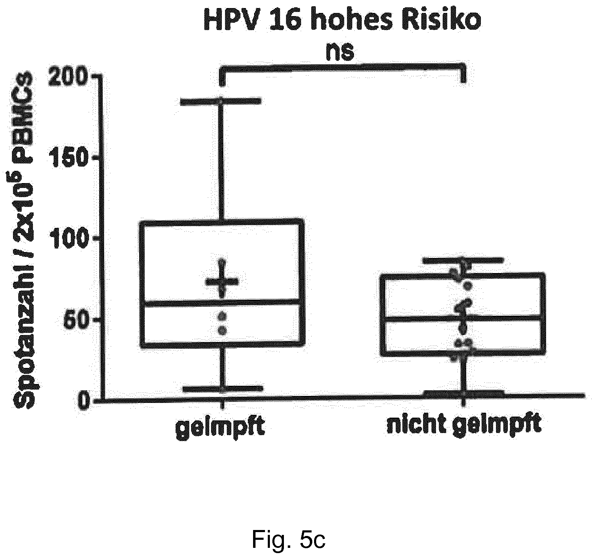

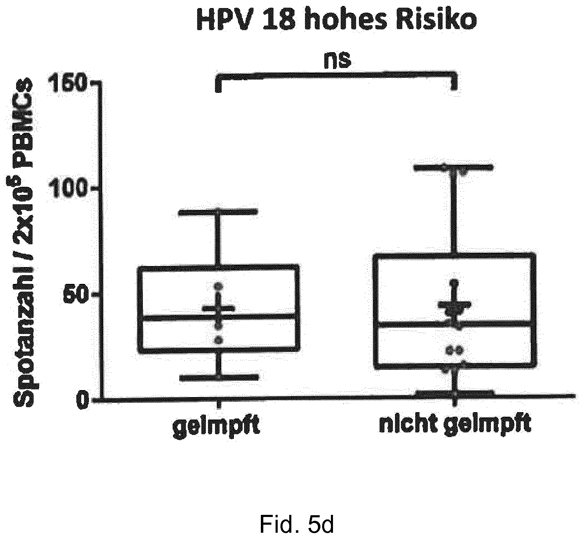

Die Figuren 5a-d zeigen grafisch eine Übersicht der Ergebnisse von 6 geimpften und 15 nicht geimpften Spendern vergleichend aufgetragen in einem Box-Whisker-Plot (Minimum bis Maximum, alle Datenpunkte).

Durchgeführt wurden Ein-Farben-Fluoreszenz Elispot Assays, bei welchen die Bestandteile des VLP Mix einzeln eingesetzt wurden. Der durchgeführte, doppelte, beidseitige t-Test(a=0,05) zeigt als Ergebnis keine signifikanten Abweichungen (ns) der Spotanzahlen zwischen den beiden Spendergruppen unter Verwendung aller HPV Typen.

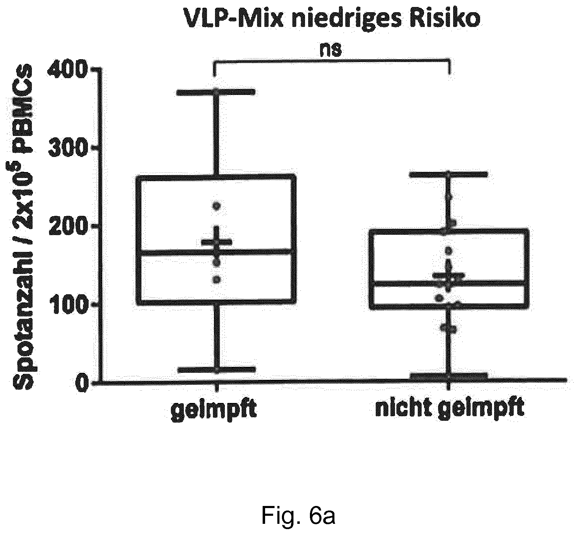

Die Figuren 6a-b zeigen grafisch eine Übersicht der Ergebnisse von 6 geimpften und 15 nicht geimpften Spendern vergleichend aufgetragen in einem Box-Whisker-Plot (Minimum bis Maximum, alle Datenpunkte).

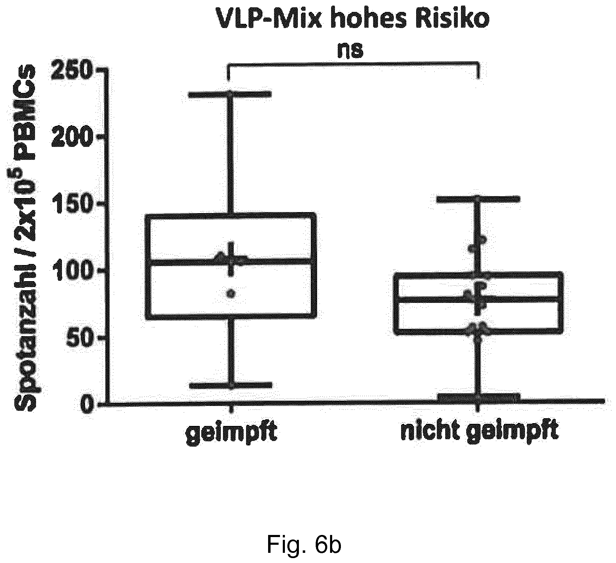

Durchgeführt wurde ein Mehr-Farben-Fluoreszenz Elispot Assay, in welchem der VLP-Mix low-Risk (Fig. 6a), bestehend aus HPV 6 und HPV 11 VLPs, welche mit Alexa-555 markiert wurden, und der VLP-Mix high-Risk (Fig. 6b), bestehend aus HPV 16 und HPV 18 VLPs, welche mit Alexa-488 markiert wurden, zusammen in einem Well inkubiert wurden.

Der durchgeführte, doppelte, beidseitige t-Test(a=0,05) zeigt als Ergebnis keine signifikanten Abweichungen (ns) der Spotanzahlen zwischen den beiden Spendergruppen unter Verwendung der low-Risk oder hight-Risk HPV Typen.

Eine mögliche Ursache der nicht signifikanten Unterschiede zwischen den beiden Spendergruppen könnte in der sehr hohen Homologie zwischen den einzelnen HPV Typen begründet sein. Es liegen keine gesicherten Daten über die Prävalenz von HPV Infektionen bei Frauen und Männern vor. Daher ist davon auszugehen, dass eine Infektion mit HPV high-Risk Typen die B-Zell-Antwort hervorruft und somit die Unterschiede in der B-Zell Antwort nicht signifikant sind.

B-Zellen erkennen dreidimensionale Epitope, welche sich durch den Austausch einzelner Aminosäuren in ihrer Konformation nur in sehr geringem Maße unterscheiden können und somit immer noch erkannt werden. Daher könnte es sich bei dem hier beobachteten Phänomen um eine Kreuzreaktion zwischen harmlosen, ubiquitären HPV Typen, welche die Haut infizieren, und den im Assay verwendeten VLPs handeln. Eine mögliche Lösung des Problems wäre, eine höhere Anzahl HPV Typen, welche alle individuell mit einem anderen Fluoreszenzfarbstoff markiert werden, einzusetzen.

Ein besonderes Augenmerk bei der validen Entwicklung immunologischer Assays liegt in der Etablierung geeigneter Kontrollen, welche die korrekte Durchführung eines jedes Assays sicherstellen. Die Negativ-Kontrolle (NC) dokumentierte dabei eine mögliche spontane Freisetzung von Antikörpern des Isotyps IgG ohne die Zugabe von Stimulantien. Durch diese Kontrolle kann eine unspezifische Stimulation der Zellen durch den Aufreinigungsprozess oder andere äußere Einflüsse erkannt und somit falsch positive Ergebnisse vermieden werden.

Die Positiv-Kontrolle hingegen dokumentierte den Einsatz der korrekten Menge an vitalen und stimulierbaren Zellen. Durch diese Kontrolle kann eine Schädigung und eine damit verbundene mögliche verringerte Reaktivität der Zellen erkannt und somit falsch negative Ergebnisse vermieden werden.

Durch die Kontrolle HPV Mix ohne das gleichzeitige Einsetzen von Zellen kann dokumentiert werden, ob und in welchem Umfang es zu einer unspezifischen Bindung der fluoreszenzmarkierten Antigene auf der Membran kommt. Eine unspezifische Bindung der markierten Antigene könnte zu falsch positiven Ergebnissen führen.

Der HPV Mix (mit Zellen) und die einzelne aufgetragenen HPV Typen dienten dem Nachweis einer antigenspezifischen Reaktion der eingesetzten Probe. Das Verhältnis der Zählwerte des HPV Mix im Vergleich zu den einzeln aufgetragenen HPV Typen diente dabei einer allgemeinen Plausibilitätsprüfung.

### BEISPIELTEIL

### 1.1 Aufreinigung der Proben

Die Isolation mononukleärer Zellen des peripheren Blutes (peripheral blood mononuclear cells, PBMC) erfolgte mittels einer isopyknischen Dichtegradientenzentrifugation mit dem Polysaccharid Ficoll-Hypaque. Dieses etablierte Standardverfahren basiert auf einem von Bøyum im Jahr 1968 erstellten Protokoll und wurde bis heute kaum verändert.

Nach der Blutentnahme wurden das antikoagulierte Vollblut und Aliquots aller weiteren Reagenzien in eine Sicherheitswerkbank eingebracht und für mindestens eine Stunde bei Raumtemperatur äquilibriert. Nachdem alle Reagenzien Raumtemperatur angenommen hatten, wurde das mit Antikoagulans versetzte Vollblut (3 Röhrchen =̂ ca. 20 ml) im Verhältnis 2:1 mit PBS (10 ml) verdünnt und durch Drehen des Röhrchens in einem flachen Winkel vorsichtig durchmischt. Anschließend wurden 15 ml Ficoll mit dem Blut-PBS-Gemisch überschichtet.

Daraufhin wurden die Röhrchen, möglichst erschütterungsfrei, in die Zentrifuge überführt und bei 1000 × g (ca. 400x g; 20 °C; 30 min) ohne die Aktivierung der Bremse, mit einer Auslaufzeit von zwei Minuten, zentrifugiert.

Die aus der Interphase entnommenen PBMCs wurden in ein 50 ml Röhrchen mit 30 ml vorgelegtem PBS überführt, um eventuell mit pipettiertes Ficoll möglichst schnell zu verdünnen, und anschließend mit PBS auf 50 ml aufgefüllt. Es folgte erneut ein Zentrifugationsschritt (700 × g; ca. 350 × g; 20 °C; 10 min), wobei nachfolgend der Überstand dekantiert und verworfen wurde. Nach zwei weiteren Waschschritten mit PBS und anschließend mit Zellkulturmedium wurden die pelletierten PBMCs, je nach Größe des Pellets, in exakt 2 oder 5 ml Medium aufgenommen und anhand einer Vitalfärbung mit Trypanblau quantifiziert.

Zur Bestimmung der Zellzahl wurden 20 µl Trypanblau in einer Mikrotiterplatte vorgelegt und 20 µl der direkt davor homogenisierten Zellsuspension hinzu pipettiert. Nach mehrmaligem Auf- und Ab-Pipettieren wurde die Zählkammer umgehend befüllt, um eine Entmischung durch sich absetzende Zellen zu vermeiden. Vor einer jeden Zählung wurde die homogene Verteilung der Zellen innerhalb der Zählkammer geprüft. War dies nicht gegeben, musste die Zellsuspension erneut homogenisiert und die Zählung wiederholt werden. Im Anschluss daran wurde die aktuelle Zellmenge berechnet und die Konzentration auf 2 × 10⁶ Zellen / ml eingestellt.

### 1.2 Vorinkubation

Um membranständige Antikörper der isolierten PBMCs zu entfernen, wurden diese vor der Verwendung nochmals behandelt. Zunächst wurde eine 30-minütige Inkubation in einem Kulturmedium bei 37 °C und anschließendes Zentrifugieren für 10 Minuten (700 × g, RT) vorgenommen. Daran schloss sich das Abgießen des Mediums und das Resuspendieren des Pellets in frischem Kulturmedium an. Es folgte eine weitere Inkubation bei 37 °C für 10 Minuten und eine weitere Zentrifugation (10 min, 700 × g, RT). Abschließend wurde der Überstand verworfen, das Pellet resuspendiert und die gewünschte Zellkonzentration eingestellt.

### 1.3 Stimulation und Detektion

Für alle durchgeführten B-Zell Elispot Assays wurden die zu untersuchenden PBMCs in einer Konzentration von 2 × 10⁶ Zellen / ml in einem Zellkulturmedium eingesetzt. Um einen Temperaturschock der Zellen und eine damit verbundene mögliche unspezifische Sekretion von Antikörpern zu vermeiden, wurden die mit Antikörpern beschichtete Mikrotiterplatte (MTP) und alle weiteren Reagenzien mindesten eine Stunde vor dem Ansetzen des Elispots aus dem Kühlschrank entnommen und bei Raumtemperatur äquilibriert.

Jedes Well wurde mit 100 µl Medium (Negativkontrolle) oder 100 µl Stimulanz (fluoreszenzmarkierte VLPs) in geeigneter Konzentration befüllt. Es folgte die Zugabe von 100 µl Zellsuspension und die Inkubation bei 37 °C für die gewünschte Dauer. Nach Ablauf dieser Zeit wurde die Platte sechs Mal mit Waschpuffer gewaschen. Da als Positivkontrolle die Gesamtzahl der IgG sekretierenden B-Zellen bestimmt wurde, wurden diese Wells gesondert behandelt. In diese Wells wurden je 100 µl Detektions-Antikörper (anti-human-IgG-550) gegeben, der mit Verdünnungspuffer verdünnt wurde. In die übrigen Wells, die fluoreszenzmarkierte VLPs beinhalteten, wurden je 100 µl WP pipettiert, um ein Austrocknen der Wells zu verhindern. Anschließend folgte eine Inkubation für zwei Stunden in einer dunklen Feuchtekammer bei Raumtemperatur. Nach fünfmaligem Waschen der Kontrollwells wurden im Folgenden 100 µl Enhancer in alle Wells pipettiert und 15 Minuten bei Raumtemperatur in einer Feuchtekammer inkubiert und die Reaktion abschließend durch Abkippen gestoppt. Nach erfolgter Trocknung ließ sich die Platte mittels eines Elispots Readers auswerten.

## Patentansprüche

1. Verfahren zur in vitro Diagnose und/oder in vitro Therapieverfolgung einer Infektion und/oder Krankheit, insbesondere Infektionskrankheit, und/oder Tumorerkrankung und/oder Autoimmunerkrankung und/oder Allergie und/oder zur Impfkontrolle und/oder zum Nachweis einer Kreuzprotektivität, wobei das Verfahren folgende Schritte aufweist:
(a) Bereitstellen
- einer Probe, welche Antikörper sezernierende Zellen enthält,
- eines Trägers mit einer Oberfläche, an welcher Anti-Ig-Antikörper immobilisiert sind, und
- einer Vielzahl unterschiedlicher Antigene aus verschiedenen infektiösen Pathogenen und/oder verschiedenen Tumorantigenen und/oder verschiedenen Autoimmun-Antigenen und/oder verschiedenen Allergenen, wobei die Antigene jeweils unterschiedlich markiert sind,
(b) Inkontaktbringen der Trägeroberfläche mit der Vielzahl unterschiedlicher Antigene sowie der Probe unter Bedingungen, welche dazu geeignet sind, dass die Antikörper sezernierenden Zellen durch die Antigene stimuliert werden und Antikörper, welche durch die stimulierten Antikörper sezernierenden Zellen produziert werden, an die immobilisierten Anti-Ig-Antikörper der Trägeroberfläche binden und die Antigene an Antikörper binden, welche für die Antigene spezifisch sind, und
(c) Detektieren von markierten Antigenen, welche an der Trägeroberfläche abgefangen wurden, wodurch die An- oder Abwesenheit von in der Probe enthaltenen Antikörper sezernierenden Zellen, welche für die Antigene spezifisch sind, detektiert wird.

2. Verfahren nach Anspruch 1, **dadurch gekennzeichnet, dass** beim Durchführen von Schritt (b) zunächst die Trägeroberfläche mit der Vielzahl unterschiedlicher Antigene in Kontakt gebracht und anschließend die Probe zu der Trägeroberfläche und der Vielzahl unterschiedlicher Antigene hinzugegeben wird.

3. Verfahren nach Anspruch 1 oder 2, **dadurch gekennzeichnet, dass** als Probe eine Probe, enthaltend mononukleäre Zellen, insbesondere B-Zellen, einer Körperflüssigkeit, insbesondere des peripheren Blutes, und/oder eines Körpergewebes verwendet wird.

4. Verfahren nach Anspruch 3, **dadurch gekennzeichnet, dass** die mononukleären Zellen, insbesondere B-Zellen, vor Durchführen von Schritt (b), vorzugsweise mittels Dichtegradientenzentrifugation oder mittels magnetischer Zellseparation (Magnetic Activated Cell Sorting, MACS), isoliert werden.

5. Verfahren nach Anspruch 3 oder 4, **dadurch gekennzeichnet, dass** zellgebundene und/oder membranständige Antikörper, insbesondere B-Zell-Rezeptoren, der mononukleären Zellen, insbesondere B-Zellen, vor Durchführen von Schritt (b) entfernt werden.

6. Verfahren nach Anspruch 5, **dadurch gekennzeichnet, dass** zum Entfernen der zellmenbrangebundenen Antikörper, insbesondere B-Zell-Rezeptoren, folgende Schritte durchgeführt werden:
- Inkubieren der mononukleären Zellen, insbesondere der B-Zellen, in einem Zellkulturmedium, bevorzugt über einen Zeitraum von 10 min bis 60 min, insbesondere 20 min bis 40 min, bevorzugt 30 min, und/oder bei einer Temperatur von 20 °C bis 40 °C, insbesondere 30 °C bis 40 °C, bevorzugt 37 °C,
- Zentrifugieren der inkubierten mononukleären Zellen, insbesondere der B-Zellen, und des Zellkulturmediums, bevorzugt mit einer Rotationsgeschwindigkeit von 400 × g bis 1000 × g, insbesondere 700 × g, und/oder über einen Zeitraum von 5 min bis 15 min, insbesondere 10 min, und/oder bei einer Temperatur von 18 °C bis 25 °C, insbesondere 20 °C,
- Abtrennen des Zellkulturmediums nach dem Zentrifugieren unter Erhalt eines Zellpellets,
- Resuspendieren des Zellpellets und Inkubieren der Zellen in einem weiteren Zellkulturmedium, bevorzugt über einen Zeitraum von 5 min bis 15 min, insbesondere 10 min, und/oder bei einer Temperatur von 30 °C bis 40 °C, insbesondere 37 °C,
- Zentrifugieren der inkubierten Zellen und des weiteren Zellkulturmediums, bevorzugt mit einer Rotationsgeschwindigkeit von 400 × g bis 1000 × g, insbesondere 700 × g, und/oder über einen Zeitraum von 5 min bis 15 min, insbesondere 10 min, und/oder bei einer Temperatur von 18 °C bis 25 °C, insbesondere 20 °C, und
- Abtrennen des weiteren Zellkulturmediums unter Erhalt eines weiteren Zellpellets, wobei Zellen des weiteren Zellpellets eine reduzierte Anzahl an membranbeständigen Antikörpern, insbesondere B-Zell-Rezeptoren, aufweisen.

7. Verfahren nach einem der vorhergehenden Ansprüche, **dadurch gekennzeichnet, dass** als Vielzahl unterschiedlicher Antigene eine Vielzahl unterschiedlicher Antigene verwendet wird, wobei die Antigene jeweils mit unterschiedlichen Farbstoffen, insbesondere unterschiedlichen Fluoreszenzfarbstoffen, vorzugsweise ausgewählt aus der Gruppe bestehend aus Cyanine, Fluorescein, Rhodamine, Curamine und Xanthene, markiert sind.

8. Verfahren nach einem der vorhergehenden Ansprüche, **dadurch gekennzeichnet, dass** der Schritt (b) über einen Zeitraum von 30 min bis 5 Tagen, insbesondere 30 min bis 48 h, vorzugsweise 30 min bis 24 h, und/oder bei einer Temperatur von 20 °C bis 40 °C, insbesondere 30 °C bis 40 °C, bevorzugt 35 °C bis 40 °C, durchgeführt wird.

9. Verfahren nach einem der vorhergehenden Ansprüche, **dadurch gekennzeichnet, dass** die Antigene beim Durchführen von Schritt (c) durch das Vorliegen eines Spots oder einer Vielzahl von Spots auf der Trägeroberfläche detektiert werden.

10. Verfahren nach einem der vorhergehenden Ansprüche, **dadurch gekennzeichnet, dass** als Vielzahl von Pathogenen unterschiedliche Subtypen desselben Pathogens und/oder als Vielzahl unterschiedlicher Antigene Varianten desselben Antigens verwendet werden.

11. Verfahren nach einem der vorhergehenden Ansprüche, **dadurch gekennzeichnet, dass** die Vielzahl unterschiedlicher Antigene ausgewählt wird aus verschiedenen infektiösen Pathogenen, welche ausgewählt sind aus der Gruppe bestehend aus Papilomaviridae, humane Papillomaviren, Herpesviridae, Herpes-simplex-Viren, Hepatitisviren, humane Immundefizienz-Viren, Influenzaviren, Parainfluenzaviren, Mumpsvirus, Masernvirus, Rötelnvirus, Parvovirus B19, Norovirus, Rotavirus, Polioviren, Coxsackieviren, Rhinoviren, Enteroviren, Adenoviren, Metapneumovirus, Mycobacterium-tuberculosis-Komplex, nichttuberkulöse Mykobakterien, Borrelien, Suptypen der genannten Pathogene und Kombinationen von wenigstens zwei der genannten Pathogene, insbesondere von wenigstens zwei Suptypen der genannten Pathogene.

12. Verfahren nach einem der vorgehenden Ansprüche, insbesondere nach Anspruch 9, **dadurch gekennzeichnet, dass** die Antigene, insbesondere der Spot bzw. die Vielzahl von Spots, beim Durchführen von Schritt (c) mittels Lichtmikroskopie, Fluoreszenzmikroskopie oder mittels eines Fluoreszenz-Messgeräts detektiert werden.

13. Verfahren nach einem der vorhergehenden Ansprüche, **dadurch gekennzeichnet, dass** ferner als Positivkontrolle die Gesamtzahl der in der Probe enthaltenen Antikörper sezernierenden Zellen gemessen wird, vorzugsweise mittels eines gegen den Isotyp der sezernierten Antikörper gerichteten Detektionsantikörpers.

14. Verfahren nach einem der vorhergehenden Ansprüche, **dadurch gekennzeichnet, dass** ferner als Negativkontrolle in Abwesenheit der Antigene eine Produktion von Antikörpern durch die Antikörper sezernierenden Zellen und/oder in Abwesenheit der Antikörper sezernierenden Zellen markierte Antigene in einem Zellkulturmedium gemessen werden.

15. Verwendung eines Kits, aufweisend räumlich voneinander getrennt
- einen Träger mit einer Oberfläche, an welcher Anti-Ig-Antikörper immobilisiert sind, und
- eine Vielzahl unterschiedlicher Antigene aus verschiedenen infektiösen Pathogenen und/oder verschiedenen Tumorantigenen und/oder verschiedenen Autoimmun-Antigenen und/oder verschiedenen Allergenen, wobei die Antigene jeweils unterschiedlich markiert sind,
oder
- einen Träger mit einer Oberfläche,
- Anti-Ig-Antikörper zur Immobilisierung an einer Oberfläche des Trägers und
- eine Vielzahl unterschiedlicher Antigene aus verschiedenen infektiösen Pathogenen und/oder verschiedenen Tumorantigenen und/oder verschiedenen Autoimmun-Antigenen und/oder verschiedenen Allergenen, wobei die Antigene jeweils unterschiedlich markiert sind,
oder
- einen Träger mit einer Oberfläche und
- Anti-Ig-Antikörper zur Immobilisierung an einer Oberfläche des Trägers,
oder
- einen Träger mit einer Oberfläche und
- eine Vielzahl unterschiedlicher Antigene aus verschiedenen infektiösen Pathogenen und/oder verschiedenen Tumorantigenen und/oder verschiedenen Autoimmun-Antigenen und/oder verschiedenen Allergenen, wobei die Antigene jeweils unterschiedlich markiert sind,
oder
- Anti-Ig-Antikörper zur Immobilisierung an einer Oberfläche des Trägers und
- eine Vielzahl unterschiedlicher Antigene aus verschiedenen infektiösen Pathogenen und/oder verschiedenen Tumorantigenen und/oder verschiedenen Autoimmun-Antigenen und/oder verschiedenen Allergenen, wobei die Antigene jeweils unterschiedlich markiert sind,
zur Durchführung eines Verfahrens nach einem der vorhergehenden Ansprüche.

## Claims

1. A method for in vitro diagnosis and/or in vitro therapy tracking of an infection and/or disease, in particular infectious disease, and/or tumour disease and/or autoimmune disease and/or allergy and/or for vaccination monitoring and/or for the detection of cross-protectivity, wherein the method includes the following steps:
(a) providing
- a sample which contains antibody-secreting cells,
- a carrier with a surface on which anti-Ig antibodies are immobilised, and
- a plurality of different antigens from various infectious pathogens and/or various tumour antigens and/or various autoimmune antigens and/or various allergens, wherein the antigens are in each case differently labelled,
(b) contacting the carrier surface with the plurality of different antigens and the sample under conditions which are suitable for the antibody-secreting cells to be stimulated by the antigens and for antibodies which are produced by the antibody-secreting cells to bind to the immobilised anti-Ig antibodies of the carrier surface and for the antigens to bind to antibodies which are specific for the antigens, and
(c) detecting labelled antigens which have been captured on the carrier surface, whereby the presence or absence of antibody-secreting cells contained in the sample which are specific for the antigens is detected.

2. A method according to Claim 1, **characterised in that**, on performance of step (b), the carrier surface is firstly contacted with the plurality of different antigens and the sample is then added to the carrier surface and the plurality of different antigens.

3. A method according to Claim 1 or 2, **characterised in that** the sample used is a sample containing mononuclear cells, in particular B cells, of a body fluid, in particular of peripheral blood, and/or of a body tissue.

4. A method according to Claim 3, **characterised in that** the mononuclear cells, in particular B cells, are isolated prior to performing step (b), preferably by way of density gradient centrifugation or by way of magnetic cell separation (Magnetic Activated Cell Sorting, MACS).

5. A method according to Claim 3 or 4, **characterised in that** cell-bound and/or membrane-located antibodies, in particular B cell receptors, of the mononuclear cells, in particular B cells, are removed prior to performing step (b).

6. A method according to Claim 5, **characterised in that** the following steps are performed to remove the cell membrane-bound antibodies, in particular B cell receptors:
- incubating the mononuclear cells, in particular the B cells, in a cell culture medium, preferably over a period of 10 min to 60 min, in particular 20 min to 40 min, preferably 30 min, and/or at a temperature of 20°C to 40°C, in particular 30°C to 40°C, preferably 37°C,
- centrifuging the incubated mononuclear cells, in particular the B cells, and the cell culture medium, preferably at a rotational speed of 400xg to 1000xg, in particular 700xg, and/or over a period of 5 min to 15 min, in particular 10 min, and/or at a temperature of 18°C to 25°C, in particular 20°C,
- separating the cell culture medium after centrifugation to obtain a cell pellet,
- resuspending the cell pellet and incubating the cells in a further cell culture medium, preferably over a period of 5 min to 15 min, in particular 10 min, and/or at a temperature of 30°C to 40°C, in particular 37°C,
- centrifuging the incubated cells and the further cell culture medium, preferably at a rotational speed of 400xg to 1000xg, in particular 700xg, and/or over a period of 5 min to 15 min, in particular 10 min, and/or at a temperature of 18°C to 25°C, in particular 20°C, and
- separating the further cell culture medium to obtain a further cell pellet, wherein cells of the further cell pellet have a reduced number of membrane-located antibodies, in particular B cell receptors.

7. A method according to one of the preceding claims, **characterised in that** the plurality of different antigens used is a plurality of different antigens, wherein the antigens are in each case labelled with different dyes, in particular different fluorescent dyes, preferably selected from the group consisting of cyanines, fluorescein, rhodamines, curamines and xanthenes.

8. A method according to one of the preceding claims, **characterised in that** step (b) is performed over a period of 30 min to 5 days, in particular 30 min to 48 h, preferably 30 min to 24 h, and/or at a temperature of 20°C to 40°C, in particular 30°C to 40°C, preferably 35°C to 40°C.

9. A method according to one of the preceding claims, **characterised in that** the antigens are detected on performance of step (c) by the presence of a spots or a plurality of spots on the carrier surface.

10. A method according to one of the preceding claims, **characterised in that** the plurality of pathogens used are different subtypes of the same pathogens and/or the plurality of different antigens used are variants of the same antigen.

11. A method according to one of the preceding claims, **characterised in that** the plurality of different antigens are selected from various infection pathogens which are selected from the group consisting of Papillomaviridae, human papillomaviruses, Herpesviridae, herpes simplex viruses, hepatitis viruses, human immunodeficiency viruses, influenza viruses, parainfluenza viruses, mumps virus, measles virus, rubella virus, parvovirus B19, norovirus, rotavirus, polioviruses, coxsackieviruses, rhinoviruses, enteroviruses, adenoviruses, metapneumovirus, Mycobacterium tuberculosis complex, nontuberculous mycobacteria, Borrelia, subtypes of said pathogens and combinations of at least two of the stated pathogens, in particular of at least two subtypes of the stated pathogens.

12. A method according to one of the preceding claims, in particular according to Claim 9, **characterised in that** the antigens, in particular the spot or the plurality of spots, are detected on performance of step (c) by way of light microscopy, fluorescence microscopy or by way of a fluorescence measuring instrument.

13. A method according to one of the preceding claims, **characterised in that** the total number of antibody-secreting cells present in the sample is measured as a positive control, preferably by way of a detection antibody directed against the isotype of the secreted antibody.

14. A method according to one of the preceding claims, **characterised in that**, as a negative control, production of antibodies by the antibody-secreting cells is furthermore measured in the absence of the antigens and/or labelled antigens are furthermore measured in a cell culture medium in the absence of antibody-secreting cells.

15. Use of a kit including, spatially separate from one another:
- a carrier with a surface on which anti-Ig antibodies are immobilised, and
- a plurality of different antigens from various infectious pathogens and/or various tumour antigens and/or various autoimmune antigens and/or various allergens, wherein the antigens are in each case differently labelled,
or
- a carrier with a surface,
- anti-Ig antibodies for immobilisation on a surface of the carrier and
- a plurality of different antigens from various infectious pathogens and/or various tumour antigens and/or various autoimmune antigens and/or various allergens, wherein the antigens are in each case differently labelled,
or
- a carrier with a surface and
- anti-Ig antibodies for immobilisation on a surface of the carrier,
or
- a carrier with a surface and
- a plurality of different antigens from various infectious pathogens and/or various tumour antigens and/or various autoimmune antigens and/or various allergens, wherein the antigens are in each case differently labelled,
or
- anti-Ig antibodies for immobilisation on a surface of the carrier and
- a plurality of different antigens from various infectious pathogens and/or various tumour antigens and/or various autoimmune antigens and/or various allergens, wherein the antigens are in each case differently labelled,
for performing a method according to one of the preceding claims.

## Revendications

1. Procédé de diagnostic in vitro et/ou de suivi de thérapie in vitro d'une infection et/ou d'une maladie, en particulier d'une maladie infectieuse, et/ou d'une maladie tumorale et/ou autoimmune et/ou d'une allergie et/ou de contrôle de la vaccination et/ou de vérification d'une protection croisée, ledit procédé présentant les étapes suivantes :
(a) Mise à disposition
- d'un échantillon contenant des cellules sécrétrices d'anticorps,
- d'un support avec une surface sur laquelle sont immobilisés des anticorps anti-Ig, et
- d'une pluralité d'antigènes différents provenant de différents agents pathogènes infectieux et/ou de différents antigènes tumoraux et/ou de différents antigènes autoimmuns et/ou de différents allergènes, les antigènes étant chacun marqués différemment,
(b) Mise en contact de la surface du support avec la pluralité d'antigènes différents ainsi qu'avec l'échantillon dans des conditions adaptées pour que les cellules sécrétrices d'anticorps soient stimulées par les antigènes et que les anticorps produits par les cellules sécrétrices d'anticorps stimulées se lient aux anticorps anti-Ig immobilisés de la surface du support et que les antigènes se lient aux anticorps spécifiques aux antigènes, et
(c) Détection des antigènes marqués qui ont été capturés à la surface du support et permettant de déterminer la présence ou l'absence de cellules sécrétrices d'anticorps spécifiques à l'antigène dans l'échantillon.

2. Procédé selon la revendication 1, **caractérisé en ce que**, lors de la réalisation de l'étape (b), la surface du support est tout d'abord mise en contact avec la pluralité d'antigènes différents avant que l'échantillon ne soit ajouté à la surface du support et à la pluralité d'antigènes différents.

3. Procédé selon la revendication 1 ou 2, **caractérisé en ce qu'**est utilisé comme échantillon un échantillon contenant des cellules mononucléaires, en particulier des cellules B, d'un liquide corporel, en particulier du sang périphérique, et/ou d'un tissu corporel.

4. Procédé selon la revendication 3, **caractérisé en ce que** les cellules mononucléaires, en particulier les cellules B, sont isolées avant la réalisation de l'étape (b), de préférence par centrifugation en gradient de densité ou par tri cellulaire magnétique (Magnetic Activated Cell Sorting, MACS).

5. Procédé selon la revendication 3 ou 4, **caractérisé en ce que** les anticorps liés aux cellules et/ou membranaires, en particulier les récepteurs de cellules B, des cellules mononucléaires, en particulier des cellules B, sont éliminés avant la réalisation de l'étape (b).

6. Procédé selon la revendication 5, **caractérisé par** la réalisation des étapes suivantes pour l'élimination des anticorps liés aux membranes cellulaires, en particulier aux récepteurs des cellules B :
- Incubation des cellules mononucléaires, en particulier des cellules B, dans un milieu de culture cellulaire, de préférence pour une durée de 10 min à 60 min, en particulier de 20 min à 40 min, de préférence de 30 min, et/ou à une température de 20 °C à 40 °C, en particulier de 30 °C à 40 °C, de préférence de 37 °C,
- Centrifugation des cellules mononucléaires incubées, en particulier des cellules B, et du milieu de culture cellulaire, de préférence à une vitesse de rotation de 400 × g à 1000 × g, en particulier de 700 × g, et/ou pour une durée de 5 min à 15 min, en particulier de 10 min, et/ou à une température de 18 °C à 25 °C, en particulier de 20 °C,
- Séparation du milieu de culture cellulaire après la centrifugation, avec obtention d'un culot de cellules,
- Remise en suspension du culot de cellules et incubation des cellules dans un autre milieu de culture cellulaire, de préférence pour une durée de 5 min à 15 min, en particulier de 10 min, et/ou à une température de 30 °C à 40 °C, en particulier de 37 °C,
- Centrifugation des cellules incubées et de l'autre milieu de culture cellulaire, de préférence à une vitesse de rotation de 400 × g à 1000 × g, en particulier de 700 × g, et/ou pour une durée de 5 min à 15 min, en particulier de 10 min, et/ou à une température de 18 °C à 25 °C, en particulier de 20 °C, et
- Séparation de l'autre milieu de culture cellulaire avec obtention d'un autre culot de cellules, les cellules de cet autre culot présentant un nombre réduit d'anticorps membranaires, en particulier de récepteurs de cellules B.

7. Procédé selon l'une des revendications précédentes, **caractérisé en ce qu'**est utilisée en tant que pluralité d'antigènes différents une pluralité d'antigènes différents, les antigènes étant respectivement marqués par des colorants différents, en particulier des colorants fluorescents différents, de préférence choisis dans le groupe constitué par les cyanines, la fluorescéine, les rhodamines, les curamines et les xanthènes.

8. Procédé selon l'une des revendications précédentes, **caractérisé en ce que** l'étape (b) est réalisée sur une durée de 30 min à 5 jours, en particulier de 30 min à 48 h, de préférence de 30 min à 24 h, et/ou à une température de 20 °C à 40 °C, en particulier de 30 °C à 40 °C, de préférence de 35 °C à 40 °C.

9. Procédé selon l'une des revendications précédentes, **caractérisé en ce que**, lors de la réalisation de l'étape (c), les antigènes sont détectés par la présence d'un point (spot) ou d'une pluralité de points sur la surface du support.

10. Procédé selon l'une des revendications précédentes, **caractérisé en ce que** sont utilisés en tant que pluralité d'agents pathogènes différents sous-types du même agent pathogène et/ou en tant que pluralité d'antigènes différents des variantes du même antigène.

11. Procédé selon l'une des revendications précédentes, **caractérisé en ce que** la pluralité d'antigènes différents est choisie parmi différents agents pathogènes infectieux qui sont sélectionnés dans le groupe composé des Papillomaviridae, des papillomavirus humains, des Herpesviridae, des virus Herpes simplex, des virus de l'hépatite, des virus de l'immunodéficience humaine, des virus influenza, des virus para-influenza, du virus des oreillons, du virus de la rougeole, du virus de la rubéole, du parvovirus B19, du norovirus, du rotavirus, des virus de la polio, des virus Coxsackie, des rhinovirus, des entérovirus, des adénovirus, du métapneumovirus, du complexe Mycobacterium tuberculosis, des mycobactéries non tuberculeuses, des Borrelia, des sous-types des agents pathogènes désignés et des combinaisons d'au moins deux des agents pathogènes désignés, en particulier d'au moins deux sous-types des agents pathogènes désignés.

12. Procédé selon l'une des revendications précédentes, en particulier selon la revendication 9, **caractérisé en ce que**, lors de la réalisation de l'étape (c), les antigènes, en particulier le point ou la pluralité de points, sont détectés par microscopie optique, par microscopie à fluorescence ou au moyen d'un appareil de mesure à fluorescence.

13. Procédé selon l'une des revendications précédentes, **caractérisé en ce qu'**est en outre mesuré, à titre de contrôle positif, le nombre total de cellules sécrétrices d'anticorps contenues dans l'échantillon, de préférence au moyen d'un anticorps de détection dirigé contre l'isotype des anticorps sécrétés.

14. Procédé selon l'une des revendications précédentes, **caractérisé en ce que** sont en outre mesurés, à titre de contrôle négatif, en l'absence d'antigènes, une production d'anticorps par les cellules sécrétrices d'anticorps et/ou, en l'absence de cellules sécrétrices d'anticorps, des antigènes marqués dans un milieu de culture cellulaire.

15. Utilisation d'un ensemble comprenant dans des espaces séparés
- un support avec une surface sur laquelle sont immobilisés des anticorps anti-Ig, et
- une pluralité d'antigènes différents provenant de différents agents pathogènes infectieux et/ou de différents antigènes tumoraux et/ou de différents antigènes auto-immuns et/ou de différents allergènes, les antigènes étant chacun marqués différemment,
ou
- un support avec une surface,
- des anticorps anti-Ig pour l'immobilisation sur une surface du support, et
- une pluralité d'antigènes différents provenant de différents agents pathogènes infectieux et/ou de différents antigènes tumoraux et/ou de différents antigènes auto-immuns et/ou de différents allergènes, les antigènes étant chacun marqués différemment,
ou
- un support avec une surface et
- des anticorps anti-Ig pour l'immobilisation sur une surface du support,
ou
- un support avec une surface et
- une pluralité d'antigènes différents provenant de différents agents pathogènes infectieux et/ou de différents antigènes tumoraux et/ou de différents antigènes auto-immuns et/ou de différents allergènes, les antigènes étant chacun marqués différemment,
ou
- des anticorps anti-Ig pour l'immobilisation sur une surface du support, et
- une pluralité d'antigènes différents provenant de différents agents pathogènes infectieux et/ou de différents antigènes tumoraux et/ou de différents antigènes auto-immuns et/ou de différents allergènes, les antigènes étant chacun marqués différemment,
pour la réalisation d'un procédé selon l'une des revendications précédentes.
